# EUROPEAN PATENT APPLICATION

(11) **EP 4 385 525 A1**
(43) Date of publication of application: **19.06.2024**
(21) Application number: 22855938.1
(22) Date of filing: 07.03.2022
(51) Int. Cl.: A61K 49/18

(54) **NANOSTRUCTURE EXCRETED IN URINE THROUGH KIDNEY WITHOUT BEING PHAGOCYTOSED AND/OR METABOLIZED BY MACROPHAGE AFTER IN VIVO INJECTION**

(30) Priority: 09.08.2021 KR 20210104405
(71) Applicant: Inventera Inc., Seoul 06588 (KR)
(72) Inventor: SHIN, Tae-Hyun, Uiwang-si Gyeonggi-do 16003 (KR)
(74) Representative: Schiweck Weinzierl Koch Patentanwälte Partnerschaft mbB
(86) International application number: PCT/KR2022/003154
(87) International publication number: WO 2023/017936

(57) **Abstract**

The present invention relates to nanostructures that, after in vivo administration, are excreted in the urine via the kidneys without being phagocytosed by macrophages and/or metabolized, and their use as pharmaceutical compositions.

A nanostructure for in vivo administration according to the present invention comprises (i) a spherical core formed by crosslinking two or three dextran molecules with an average molecular weight of 10,000 Da or less using a crosslinker and (ii) a discontinuous shell with divalent or trivalent iron ions coordinationally bonded to crosslinker-derived hydrophilic groups on the surface of the spherical core; and
(iii) a mass ratio of dextran to iron ranging from 100 : 2 to 100 : 10, with a crosslinker substitution ratio adjusted to 10% to 50% of the total number of functional groups on the dextran, while 20% to 50% of the crosslinker does not participate in crosslinking at the terminal end, resulting in the nanostructure having the charge ranging from -20 mV to 0 mV;
(iv) wherein only 20% to 80% of the crosslinker-derived hydrophilic groups externally exposed on the surface of the spherical core bind iron ions, while the remainder do not bind iron ions and are exposed to an aqueous environment.

## Description

### TECHNICAL FIELD

The present invention relates to nanostructures that, after in vivo administration, are excreted in the urine via the kidneys without being phagocytosed by macrophages and/or metabolized, and their use as pharmaceutical compositions.

### BACKGROUND ART

An essential characteristic of nanoparticles for in vivo use is their biocompatibility or bioinertness, which means that they should not cause immune rejection, such as allergies, when injected into the body, and should not be toxic.

When the main component of nanoparticles is metal and the metal component is toxic to the body, it is mostly due to the oxidation of some metal atoms into cations, which can damage cell membranes in vivo. The case of silver is typical, where the Ag(0) state may not have any effect in vivo, but when it is easily etched by electrolyte components in the body, silver cations (Ag+) and excess oxides are generated, which can destroy cell membranes.

Inorganic nanoparticles, such as metal oxides, quantum dots, and noble metals, exhibit unique optical, magnetic, and electrical properties that set them apart from organic nanoparticles. These physicochemical properties can be harnessed to realize medically useful functions, such as the precise observation, regulation, and control of biological phenomena at the molecular level. Despite this potential, in vivo injected inorganic nanoparticles are recognized as invaders by the immune system, phagocytosed by macrophages, and accumulated in organs, where they are not easily excreted. If inorganic nanoparticles are composed of heavy metals, such as quantum dots, they can be toxic if they are not excreted and degraded in the body. In order for nanoparticles to be used as a contrast agent for diagnosing diseases in the body, it is necessary to manufacture them in a form that is safe and can be excreted without being metabolized by the body after use.

Magnetic resonance imaging (MRI) is a method of obtaining anatomical, physiological, and biochemical information of the body by using the phenomenon of relaxation of the nuclear spin of hydrogen atoms in water in a magnetic field, and is currently one of the imaging diagnostic devices that can non-invasively and real-time image the body organs of living people or animals.

In order to make MRI more precise and versatile in life sciences and medicine, substances are injected externally to increase image contrast. These substances are called contrast agents and are superparamagnetic or paramagnetic, and can be used to contrast the signals of areas that need to be seen in an MRI image so that they can be clearly distinguished. Contrast between tissues in an MRI image is a phenomenon that occurs because the spin of the hydrogen atoms in the water in the tissue, which is excited by external energy and then returns to equilibrium, is different in different tissues, and contrast agents affect this relaxation, causing differences in relaxation between tissues and causing changes in the MRI signal, making the contrast between tissues clearer.

Contrast agents are used to enhance the imaging signal of certain organs and tissues by increasing or decreasing their signal relative to the surrounding area. A contrast agent that makes the image signal of the body part to be imaged relatively higher than the surrounding area is called a "positive" contrast agent (T1 contrast agent), and a contrast agent that makes the image signal relatively lower than the surrounding area is called a "negative" contrast agent (T2 contrast agent). More specifically, MRI contrast agents are divided into T1 contrast agents, which utilize the high spin of paramagnetic materials, and T2 contrast agents, which utilize the magnetic inhomogeneity around ferromagnetic or superparamagnetic materials.

T1 contrast agents are those that are associated with longitudinal relaxation. This longitudinal relaxation is a process in which the magnetisation component (Mz) in the Z-axis direction of the spin absorbs the RF energy shock from the X-axis, aligns with the Y-axis of the X-Y plane, releases the energy to the outside, and returns to its original value, and this phenomenon is called "T1 relaxation". The time until Mz returns to 63% of its initial value is called the "T1 relaxation time", and the shorter the T1 relaxation, the larger the MRI signal and the brighter the MRI image.

T2 contrast agents are those that are associated with transverse relaxation. When the magnetization component Mz in the Z-axis direction of the spin absorbs the RF energy shock from the X-axis and aligns with the Y-axis in the X-Y plane, the energy decays by itself or attempts to return to its original value by decaying its own energy or releasing energy to the surrounding spins, the phenomenon that the component Mxy of the spin in the x-y plane decays exponentially with time is called "T2 relaxation". The time until Mxy decays to 37% is called the "T2 relaxation time", and the free induction decay (FID) signal measured by a receiving coil placed in the Y-axis of the X-Y plane as a function of time as Mxy decays with time is called the free induction decay (FID) signal. Tissue with a short T2 relaxation time appears dark on MRI.

To date, commercially available MRI contrast agents use paramagnetic compounds as "positive" contrast agents and superparamagnetic nanoparticles as "negative" contrast agents. Currently, iron oxide nanoparticles such as SPIO (superparamagnetic iron oxide) are used as T2 contrast agents, but T2 contrast is a negative contrast method in which the desired area is darkened compared to the surrounding area, which has the disadvantage of not having a large contrast effect, and a larger area is imaged due to the blooming effect. On the other hand, TI contrast agents have the advantage of being positive contrast and brightening the image of the desired area, and high spin material is used. For this reason, gadolinium complexes with seven valence spins in the 4f orbital are typically used.

Gadolinium-based contrast agents (GBCAs) were commercialized in the 1980s and have not undergone significant technological advances since then. Recently, the risks of gadolinium-based contrast agents have been highlighted by reports of irreversible skin and organ hardening (nephrogenic systemic fibrosis) due to the toxicity of free gadolinium, as well as permanent deposition of gadolinium in the brain tissue of patients who received MRI contrast agents. In addition, the prevalence and mortality of various vascular diseases in patients with chronic kidney failure is high, but GBCA is banned due to the risk of nephrogenic systemic fibrosis, so there is an urgent need to develop MRI contrast agents that can be used safely in this population.

On the other hand, iron oxide as a superparamagnetic material can be categorized into two types depending on the particle size. If the particle size is 50 nm or larger, it is called SPIO, and if it is smaller, it is called USPIO (ultrasmall superparamagnetic iron oxide). The smaller particle size of USPIO makes it less susceptible to macrophage phagocytosis in blood vessels, and its long retention time can be used to identify abnormalities in blood vessels. The injection volume is also smaller than that of SPIO, allowing for rapid infusion. Feridex and Resovist, which have been used in clinical practice, are representative examples of SPIOs. Both of these contrast agents were synthesized through a method called co-precipitation, which has the limitations of poor crystallinity, low magnetic properties, and uneven size.

Since the late 1990s, using a newly developed pyrolysis method for nanoparticle synthesis, uniform iron oxide nanoparticles with a size of 5 to 20 nm have been developed, and it has been reported that they have superior T2 contrast effects compared to nanoparticles developed by the co-immersion method. However, since T1-weighted images are more accurate than T2-weighted images with severe signal interference and are preferred in clinical practice, nanoparticles with comparable or better T1 contrast effects are needed to replace gadolinium-based contrast agents.

### DETAILED DESCRIPTION OF THE INVENTION

### TECHNICAL PROBLEM

The present invention seeks to address the challenges of iron oxide nanoparticles as magnetic resonance imaging (MRI) contrast agents, such as low T1 contrast effectiveness, long-term accumulation, and toxicity.

To this end, the present invention seeks to provide nanostructures for in vivo administration that exhibit pharmacokinetics that result in complete clearance from the body without accumulation in normal tissues in the body.

Furthermore, the present invention seeks to provide nanostructures for in vivo administration that are not phagocytosed by macrophages and are not metabolically degraded after in vivo administration and are excreted in the urine via renal filtration.

Furthermore, the present invention seeks to provide a nanostructure for in vivo administration that is absorbed by the vascular circulation after in vivo administration and, after absorption, can be collected in the urine through renal filtration without extravasation and can be reused.

### TECHNICAL SOLUTION

A first aspect of the invention provide a nanostructure for in vivo administration that, after in vivo administration, is excreted in the urine via the kidneys without being phagocytosed by macrophages and/or metabolized, comprising
(i) a spherical core formed by crosslinking two or three dextran molecules with an average molecular weight of 10,000 Da or less using a crosslinker and (ii) a discontinuous shell with divalent or trivalent iron ions coordinationally bonded to crosslinker-derived hydrophilic groups on the surface of the spherical core; and
(iii) a mass ratio of dextran to iron ranging from 100 : 2 to 100 : 10, with a crosslinker substitution ratio adjusted to 10% to 50% of the total number of functional groups on the dextran, while 20% to 50% of the crosslinker does not participate in crosslinking at the terminal end, resulting in the nanostructure having the charge ranging from -20 mV to 0 mV;
(iv) wherein only 20% to 80% of the crosslinker-derived hydrophilic groups externally exposed on the surface of the spherical core bind iron ions, while the remainder do not bind iron ions and are exposed to an aqueous environment.

A second aspect of the invention provides a pharmaceutical composition comprising a nanostructure for in vivo administration that after in vivo administration, is excreted in the urine via the kidneys without being phagocytosed by macrophages and/or metabolized,
wherein the nanostructure is a nanostructure for in vivo administration of the first aspect, comprising
(i) a spherical core formed by crosslinking two or three dextran molecules with an average molecular weight of 10,000 Da or less using a crosslinker and (ii) a discontinuous shell with divalent or trivalent iron ions coordinationally bonded to crosslinker-derived hydrophilic groups on the surface of the spherical core; and
(iii) a mass ratio of dextran to iron ranging from 100 : 2 to 100 : 10, with a crosslinker substitution ratio adjusted to 10% to 50% of the total number of functional groups on the dextran, while 20% to 50% of the crosslinker does not participate in crosslinking at the terminal end, resulting in the nanostructure having the charge ranging from -20 mV to 0 mV;
(iv) wherein only 20% to 80% of the crosslinker-derived hydrophilic groups externally exposed on the surface of the spherical core bind iron ions, while the remainder do not bind iron ions and are exposed to an aqueous environment.

Hereinafter, the present invention is described.

Nanostructures for in vivo administration according to the present invention may be administered or injected and are not limited by the method of administration as long as they can be applied in vivo.

Nanostructure for in vivo administration according to the present invention that, after in vivo administration, is excreted in the urine via the kidneys without being phagocytosed by macrophages and/or metabolized, comprising
(i) a spherical core formed by crosslinking two or three dextran molecules with an average molecular weight of 10,000 Da or less using a crosslinker and (ii) a discontinuous shell with divalent or trivalent iron ions coordinationally bonded to crosslinker-derived hydrophilic groups on the surface of the spherical core; and
(iii) a mass ratio of dextran to iron ranging from 100 : 2 to 100 : 10, with a crosslinker substitution ratio adjusted to 10% to 50% of the total number of functional groups on the dextran, while 20% to 50% of the crosslinker does not participate in crosslinking at the terminal end, resulting in the nanostructure having the charge ranging from -20 mV to 0 mV;
(iv) wherein only 20% to 80% of the crosslinker-derived hydrophilic groups externally exposed on the surface of the spherical core bind iron ions, while the remainder do not bind iron ions and are exposed to an aqueous environment.

In this form, the nanostructures of the present invention for in vivo administration, carefully designed and synthesized, are stable in buffer solutions of physiological pH and in plasma without aggregation or free iron leaching, and can exhibit compact hydrodynamic diameters smaller than the renal filtration size limit and good colloidal stability.

Non-limiting examples of hydrophilic functional groups that coordinationally bind iron ions include hydroxy, carboxylic acid, carboxylate, and amine.

After crosslinking dextran intramolecularly and intermolecularly with a crosslinker, the inventors found that divalent and trivalent iron ions coordinationally bound to the crosslinker-derived hydrophilic groups exposed on the surface of the dextran-based spherical core could produce T1 contrast effect, whereas divalent and trivalent iron ions coordinationally bound to the hydrophilic groups of the dextran itself that were not crosslinked with a crosslinker could not produce T1 contrast effect.

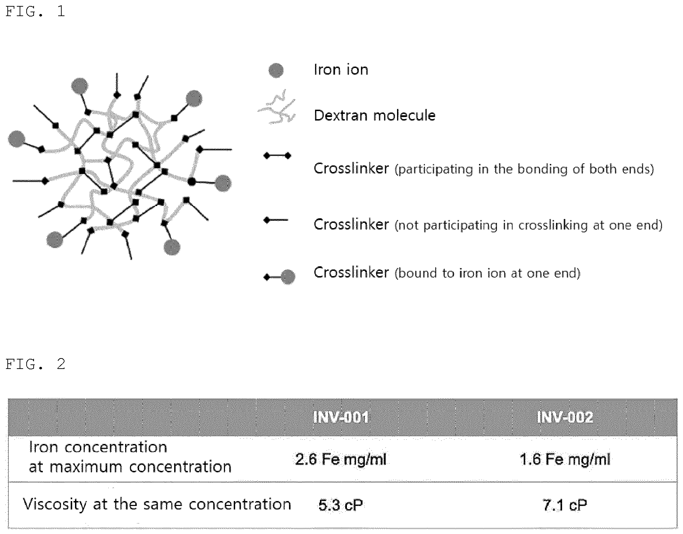

Based on this, the nanostructure for in vivo administration of the present invention are formed by crosslinker-derived hydrophilic groups exposed on the surface of a dextran-based spherical core coordinationally bonding with divalent to trivalent iron ions as ligands to form a shell composed of divalent to trivalent iron ions (FIG. 1). In this case, one to three crosslinkers exposed at the terminal end can coordinationally bind to one iron ion.

The crosslinker-derived hydrophilic functional groups can be the terminal functional groups of the crosslinker itself or modified/substituted functional groups thereof. For example, divalent or trivalent iron ions can be coordinationally linked to crosslinker-derived carboxylic acid or carboxylate groups on the surface of the dextran spherical core.

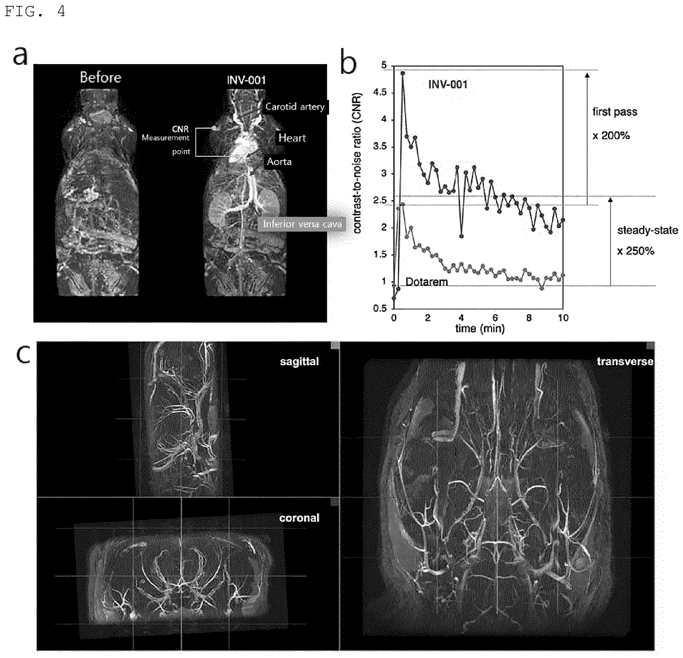

The inventors have found that the nanostructures for in vivo administration according to the present invention (Example 1) can achieve the appropriate physicochemical properties that an intravenous drug formulation should have, such as osmolarity and viscosity (FIG. 2), and have confirmed in animal studies that they can act as a T1 MRI contrast agent after intravenous injection, without leakage through the blood vessel wall, without being phagocytosed by macrophages and without being metabolized, and can be collected and recycled in the urine (FIGS. 4 to 8).

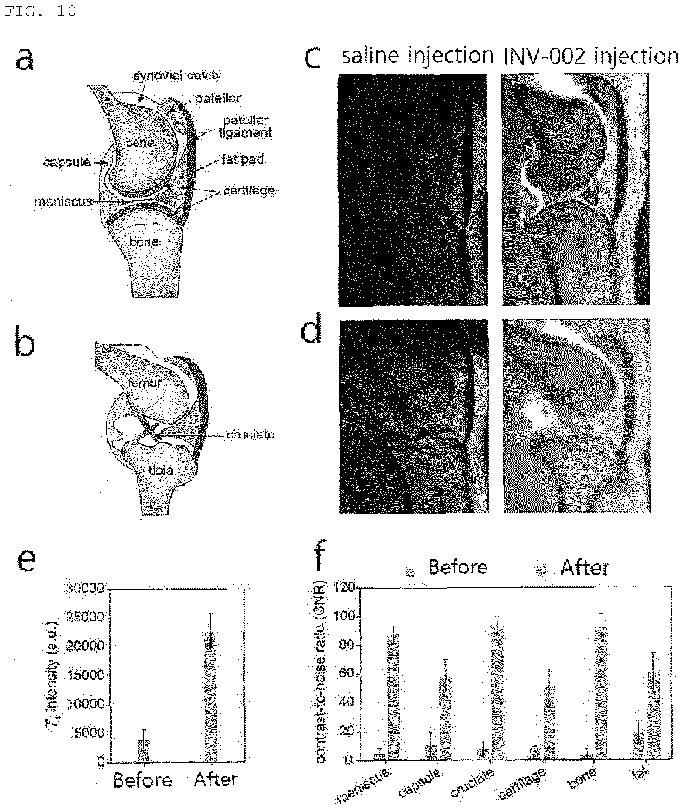

Furthermore, it was found that the nanostructures for in vivo administration (Example 2) according to the present invention are not metabolized or degraded in the body, even when administered intra-articularly or intrathecally, but are absorbed into the circulatory system (e.g., the venule) and excreted into the urine via renal filtration without leakage through the vessel wall (FIG. 10 to FIG. 16), acting as a T1 MRI contrast agent.

The present invention is based on this.

The nanostructures for in vivo administration of the present invention can be designed to have the function of a T1 MRI contrast agent that exhibits a bright signal on MRI, so that the location of the nanostructures can be tracked via MRI after in vivo administration, and thus the present invention can be used to determine whether the nanostructures are phagocytosed by macrophages, metabolized, circulated in the blood, or circulated in the lymphatic fluid after in vivo administration, delivery to the parenchyma of a cell through capillaries, accumulation in tissues, excretion through the kidneys into the urine, absorption into the vascular circulation after in vivo administration, leakage through the blood vessel wall, and collection and reusability through urine can be determined through animal studies, thereby providing customized biocompatible nanostructures that exhibit desired pharmacokinetic and pharmacodynamic properties.

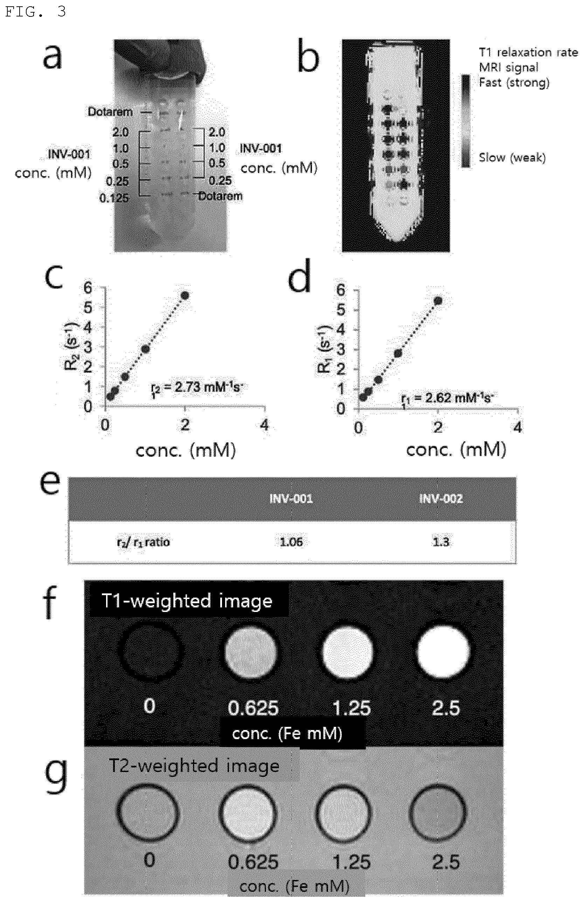

Furthermore, since the T1 MRI signal intensity exhibited by the nanostructures for in vivo administration of the present invention can be designed to be proportional to the concentration of the nanostructures, it is possible to quantify the concentration of the nanostructures from the signal in the MRI image (Example 6 and FIG. 3). Accordingly, the nanostructures for in vivo administration of the present invention can be designed to also serve as contrast agents that can provide information about the pathways by which injected nanomaterials are absorbed, distributed, metabolized, and excreted in vivo, as well as various anatomical structures and functions located along those pathways. Further, animal studies can be used to analyze the in vivo behavior of nanostructures for in vivo administration or pharmaceutical compositions containing them, depending on the site of application and/or route of administration, and for quality control to ensure that the nanostructures or pharmaceutical compositions have been manufactured with uniform quality to achieve the desired in vivo behavior. By utilizing various accumulated data for bioinformatics, it is possible to elaborately design nanostructures for in vivo administration customized to the physiology of the patient (condition and/or history), the disease, the route of administration, and/or the physiological mechanism of the drug to be delivered via the nanostructures of the present invention.

### [Method for preparation of nanostructures for in vivo administration].

The nanostructures for in vivo administration of the present invention can be provided by a preparation method comprising, as a non-limiting example, the steps below:
1^{st} step of preparing an aqueous solution of dextran;
2^{nd} step of adding an alkaline aqueous solution and an epoxide at room temperature;
3^{rd} step of adding two or more polyvalent amines at room temperature, to generate crosslinked dextran-based nanoparticles with terminal amine groups;
4^{th} step of precipitate the product;
5^{th} step of redispersing the product in water;
optionally, 6^{th} step of recovering the crosslinked dextran-based nanoparticles with terminal amine groups by dialysis;
7^{th} step of treating an organic acid anhydride to the crosslinked dextran-based nanoparticles crosslinked by a polyvalent amine group and having terminal amine groups, to substitute some or all of the terminal amine groups with carboxylic acid groups and/or carboxylate groups;
optionally, 8^{th} step of purifying a solution of crosslinked dextran-based nanoparticles with carboxylic acid and/or carboxylate functional groups, to prepare water dispersible, crosslinked dextran-based nanoparticles;
9^{th} step of adding an aqueous solution of an iron precursor to the water dispersible, crosslinked dextran-based nanoparticles prepared in the preceding step, to form a shell comprising divalent to trivalent iron ions; and
optionally, 10^{th} step of purifying or concentrating the nanostructures formed in the preceding step by ultrafiltration.

The nanostructures for in vivo administration of the present invention synthesized via the above preparation method were analyzed as shown in Example 3.

In order to prevent unintended increase in hydration size due to swelling of dextran molecules, the present invention crosslinks dextran monomers intramolecularly and/or intermolecularly in aqueous solution. It was found that when dextran is crosslinked by a crosslinker, two or three dextran molecules form a spherical core through intramolecular and intermolecular crosslinking. The spherical core may be a crosslinked dextran-based nanoparticle formed by intramolecular and/or intermolecular crosslinking with a crosslinker at the -OH functional group of the glucose building block in the dextran molecule.

Crosslinked dextran-based nanoparticles can have the same or different control over the number of dextran molecules subject to crosslinking through their synthesis conditions and/or purification.

For example, the present invention can prepare crosslinked dextran-based nanoparticles by reacting an aqueous solution of dextran or a derivative thereof with (i) an epoxide that modifies the -OH functional group site of the glucose building block and (ii) a crosslinker at room temperature without expensive catalysts via 2^{nd} and 3^{rd} steps, thereby crosslinking the -OH functional group site of the glucose building block of dextran with the crosslinker.

In 2^{nd} step, the epoxide is not limited in type, as long as it modifies the -OH functional group site of the glucose building block to make it reactive with the crosslinker, and can preferably be a halo alkyl oxirane, such as epichlorohydrin.

In 3^{rd} step, the polyvalent amine can be replaced by any crosslinker as long as it can be covalently linked to epoxide-derived functional groups that modify the -OH functional site of the glucose building block, which also falls within the scope of the present invention.

2^{nd} and 3^{rd} steps are economical because they do not require additional, expensive catalysts to crosslink the -OH functional groups of the glucose building blocks with the crosslinker.

4^{th} step can be performed by adding a large amount of organic solvent with a dielectric constant between 15 and 50.

The nanostructures for in vivo administration of the present invention can be synthesized at room temperature, atmospheric pressure, and in an aqueous phase, thus eliminating the need for a separate hydrophilization process after synthesis, which is synthetically different from conventional iron oxide-based nanoparticle T1 MRI contrast agents, which are synthesized in a high-temperature, inert atmosphere, organic solvent phase at temperatures above 200 °C and involve a hydrophilization process.

Conventional iron oxide-based nanoparticle T1 MRI contrast agents are synthesized in organic solvents and typically have hydrophobic molecules attached to their surface, and hydrophilization is essential for in vivo application. Typically, hydrophilization involves replacing hydrophobic molecules with hydrophilic molecules or adding hydrophilic molecules to hydrophobic molecules to create a bilayer, which is known to dramatically increase the hydration size of the nanomaterial relative to the nanomaterial core size. Unfortunately, the behavior of injected nanomaterials in vivo follows the hydration size and not the core size. In contrast, the nanostructures for in vivo administration of the present invention have hydration sizes and molecular weights smaller than the renal filtration size limit and can be synthesized in aqueous solution rather than organic solvents and do not require a separate hydrophilization process to be colloidal stable.

As such, the nanostructures for in vivo administration of the present invention are synthesized in aqueous solution at room temperature without surfactants and without a ligand exchange step, and their water compatibility is favorable for biological use.

Furthermore, the nanostructures for in vivo administration obtained in the above preparation method are in the form of colloids dispersed in water, which, depending on the degree of concentration, can be made isotonic without any excipients, and can be used as injectable solution obtained by themselves, e.g., through the 10^{th} step, satisfying non-pyrogenicity and sterility without any special additional processing.

### [Spherical core based on crosslinked dextran].

Dextran is a polysaccharide derived from the condensation of glucose and is a complex branched glucan, as shown in the structural formula below. It is a branched poly-α-D-glucoside of microbial origin with a predominantly C-1 → C-6 glycosidic linkage.

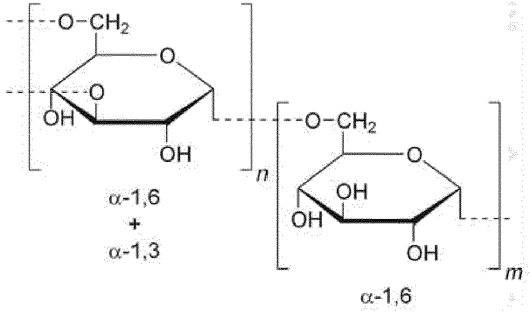

The main chain of the polymer is composed of α(1→6) glycosidic linkages between the glucose monomers, with the branches connected by α(1→3) glycosidic linkage.

Dextran was discovered as a microbial product of wine, but mass production became possible after a process using bacteria was developed. Dextran is currently produced from sucrose by certain lactic acid bacteria of the genus Lactobacillus.

Dextran is approved by the FDA as a biocompatible material.

As used herein, dextran also includes various derivatives thereof. Non-limiting examples of dextran derivatives include carboxymethyl dextran (CM dextran), dextran sulphate, and diethylaminoethyl dextran (DEAE-dextran).

The demand for dextran of various specific sizes is increasing in industrial applications. For example, dextran with a size in the range of 70,000 to 100,000 Da is used as a plasma substitute. In addition, dextran of 40,000 Da is used to improve blood flow, most likely by reducing blood viscosity and inhibiting red blood cell aggregation. Smaller dextran sulfates of about 10,000 Da are used as iron transporters or anticoagulants.

In the nanostructures for in vivo administration of the present invention, the spherical core is formed by the intra- and/or intermolecular crosslinking of complex branched dextran by crosslinkers. Complex branched dextran can form dimers or trimers through intermolecular cross-linking in aqueous solution, which can prevent unintended increase in hydration size due to swelling of dextran molecules. In the nanostructures for in vivo administration of the present invention, the spherical core formed by controlling the synthesis conditions to intramolecularly crosslink only one complex branched dextran molecule with crosslinkers also falls within the scope of the present invention.

Crosslinked dextran-based nanoparticles, in which complex branched dextran-based monomers are intramolecularly and/or intermolecularly crosslinked with crosslinkers at the -OH functional groups of the glucose building blocks in aqueous solution and compactly particulate according to the present invention, can be easily controlled to have a desired hydration size and hydration swellability, and their mobility in body fluids can be predictably controlled. For example, in the crosslinked dextran-based nanoparticles of the present invention, the crosslinker substitution ratio can be adjusted from 2% to 50% of the number of functional groups on the dextran, and 2% to 98% of the crosslinker can be adjusted such that one end is not involved in crosslinking and the other end is exposed to the outside, so that the swelling and hydration size of the crosslinked dextran-based nanoparticles can be precisely controlled as desired.

For renal excretion, the average molecular weight of the dextran used is preferably 10,000 Da or less, and the molecular weight of the spherical core formed by cross-linking the dextran molecules is 35,000 Da or less.

In particular, aqueous solutions of polymers, such as crosslinked dextran-based nanoparticles, become more viscous with increasing concentration. According to the Mark-Houwink Sakurada (MHS) equation, an expression for the molecular weight and viscosity of polymeric materials, it is known that the viscosity of a polymeric material is directly proportional to its molecular weight. Excessively high viscosity of an injectable may be associated with risks such as vessel occlusion and damage to blood vessels due to high pressure during injection. If the nanostructures for in vivo administration of the present invention are to be utilized for intravenous injection, it has been found that an appropriate viscosity for intravenous injection can be achieved by using dextran molecules with an average molecular weight of 10,000 Da or less, preferably about 5,000 Da or less, and a molecular weight of about 15,000 Da or less for the spherical core formed by crosslinking the dextran molecules (Example 5).

Furthermore, as can be seen in Examples 1-5 and FIG. 2, the smaller the average molecular weight of the dextran molecules, the higher the concentration of the nanostructures of the present invention can be prepared, and thus the viscosity of the formulation can be finely controlled by adjusting the degree of dilution, and the injection volume can be reduced for the desired dosage.

When dextran molecules having an average molecular weight of 10,000 Da or less are crosslinked with a crosslinker in an aqueous solution, two or three dextran molecules form a spherical core through intra- and intermolecular cross-linking, so that from the nanostructures of the present invention having an average molecular weight of 10,000 Da or less of the dextran molecules used, nanostructures having a molecular weight of 35,000 Da or less of the spherical core formed by cross-linking the dextran molecules and a hydrated diameter of 10 nm or less, preferably 5 nm or less, for renal excretion can be realized.

In accordance with the present invention, a dextran-based spherical core further modified with a crosslinker during a crosslinking reaction and/or the one end of the crosslinker exposed after the crosslinking reaction provides a ligand whose surface-exposed hydrophilic groups coordinationally bind iron ions.

The hydrophilic functional groups may be derived from functional groups on the dextran that did not participate in the crosslinking reaction, functional groups on the terminal end of the crosslinker that did not participate in the crosslinking reaction, and/or functional groups that further modified the terminal end of the crosslinker exposed after the crosslinking reaction.

Non-limiting examples of hydrophilic functional groups that coordinationally bind iron ions include hydroxy, carboxylic acid, carboxylate, and amine.

If the hydrophilic functional groups exposed on the surface of the nanostructures for in vivo administration of the present invention are amines, high pH toxicity may occur, which can be addressed by substituting some or all of the amine groups with carboxyl groups, methyl groups, ethyl groups, etc.

Since it is not necessary for the shell portion comprising divalent or trivalent iron ions to cover the entire surface of the crosslinked dextran-based spherical core in order for the nanostructures for in vivo administration of the present invention to serve as a T1 MRI contrast agent, the nanostructures for in vivo administration of the present invention are characterized in that some of the hydrophilic groups at one end of the crosslinker linked to the dextran are designed to be exposed to the aqueous environment without binding to the iron ions for dispersion in an injection solution and/or body fluid (FIG. 1).

The terminal hydroxy and, for example, non-coordinated carboxylate functional groups of the dextran-based spherical cores provide water solubility to the nanostructures for in vivo administration of the present invention. Thus, the hydration of hydrophilic groups exposed on the surface of the dextran-based spherical core can improve the dispersion stability in body fluids. Thereby, the dispersion stability of the nanostructures can be secured, enabling the nanostructures for in vivo administration of the present invention to be stably dispersed in body fluids without precipitation or aggregation and to exert their functions.

Thus, by varying the size and charge of the dextran in the crosslinked spherical core, the overall size and charge of the nanostructures for in vivo administration can be controlled to match the desired blood circulation time and renal excretion profile. Furthermore, by adjusting the binding ratio of externally exposed negatively charged functional groups and positively charged iron ions of the crosslinker-derived hydrophilic groups within a range of 20% to 80%, the charge of the nanostructures for in vivo administration of the present invention can be adjusted to be between -20 mV and 0 mV.

Non-limiting examples of crosslinker-derived functional groups or hydrophilic functional groups exposed on the surface of the crosslinked dextran-based spherical core include amine groups, carboxyl groups, hydroxyl groups, and/or thiol groups. Reactive functional groups such as amine, thiol, carboxyl, and hydroxyl facilitate surface modification as well as chemical binding to biopharmaceuticals or various types of small molecule drugs, such as ligands that specifically bind to receptors on specific cells, antibodies or fragments thereof, antigenic peptides, nucleic acids (such as DNA, RNA, or fragments thereof).

### [A discontinuous shell of divalent and trivalent iron ions coordinationally bonded to crosslinker-derived hydrophilic groups on the surface of a spherical core].

The nanostructures for in vivo administration of the present invention, in which divalent to trivalent iron ions are coordinationally bound to crosslinker-derived hydrophilic groups on the surface of the spherical core, are stable without free iron in buffer solutions of physiological pH and in various body fluids such as plasma and lymphatic fluid.

The nanostructures for in vivo administration of the present invention comprise a spherical core formed by crosslinking two or three dextran molecules with an average molecular weight of 10,000 Da or less; and a discontinuous shell with divalent to trivalent iron ions coordinationally bound to the crosslinker-derived hydrophilic groups on the surface of the spherical core (FIG. 1), wherein the crosslinker-derived hydrophilic groups exposed on the surface of the dextran-based spherical core are coordinationally bound to the divalent to trivalent iron ions as ligands to form a discontinuous shell comprising divalent to trivalent iron ions, wherein depending on the coverage and/or thickness of the discontinuous shell, it can be designed to function as a T1 MRI contrast agent that exhibits a bright signal in MRI images, or as a T2 MRI contrast agent as needed.

The ratio of the spin-spin relaxivity coefficient (*r*₂) to the spin-lattice relaxivity coefficient (*r*₁ ) (r₂ /r₁ ratio) is a measure of whether a contrast agent is suitable as a T1 MRI contrast agent or a T2 MRI contrast agent, with typical T1 MRI contrast agents having a *r*₂ /*r*₁ ratio of about 1 to 2, and T2 MRI contrast agents preferably having a *r*₂ /*r*₁ ratio of 5 or more.

In accordance with one embodiment of the present invention, a nanostructure for in vivo administration having a discontinuous shell with divalent or trivalent iron ions coordinationally bonded to crosslinker-derived hydrophilic groups on a spherical core surface was synthesized, and it was confirmed that it can be used as a T1 MRI contrast agent by measuring r₁ and r₂, respectively (Example 6, FIG. 3). Thus, the nanostructures for in vivo administration of the present invention having a discontinuous shell with divalent or trivalent iron ions coordinationally bonded to crosslinker-derived hydrophilic groups on the surface of the spherical core can be designed to have the function of a T1 MRI contrast agent that exhibits a bright signal in MRI images.

In addition, the discontinuous shell composed of divalent and trivalent iron ions is not coated with an additional chemical, but is exposed on its own, facilitating access to water molecules, which is more favorable for T1 MRI contrast effects such as accelerated relaxation of water molecule protons.

For example, nanostructures for in vivo administration of the present invention can be designed and synthesized to have substantially small magnetization comparable to clinical gadolinium T1 MRI contrast agents (GBCAs) and to have optimal T1 MRI contrast with an ideal low r₂ /r₁ ratio (Example 6, FIG. 3) .

Thus, the nanostructures for in vivo administration of the present invention can provide imaging to visualize the anatomical structures of microvessels, ureters, lymphatic vessels, liver, spleen, joint cavities, spinal cord cavities, and/or various organs in vivo due to their T1 MRI contrast agent properties. MRI can also reveal tissue accumulation in the body and/or leakage through the vessel wall.

According to the results of T1 MRI angiography performance analysis of a nanostructure for in vivo administration having a discontinuous shell with divalent to trivalent iron ions coordinationally bonded to crosslinker-derived hydrophilic groups on the surface of a spherical core according to the present invention (Example 7), T1 MRI contrast enhancement was observed in the carotid artery, heart, aorta, and inferior vena cava of a mouse after intravenous injection (FIG. 4(a)). The vascular system appeared brighter after injection compared to before injection, and even smaller vessels were observed compared to conventional gadolinium contrast agents (FIG. 4(c)). In addition, the contrast-to-noise ratio (CNR) measured in the cerebral cardiovascular system during the first pass was 4.87, representing a 200% improvement over gadolinium contrast agent (Dotarem), and the CNR measured in the cerebral cardiovascular system at steady-state was found to be 250% improved over Dotarem, confirming that the contrast effect of the in vivo injectable nanostructures having a discontinuous shell with divalent or trivalent iron ions coordinationally bonded to the crosslinker-derived hydrophilic groups on the surface of the spherical core according to the present invention is stronger and longer lasting than that of Dotarem (FIG. 4(b)). Thus, according to the present invention, nanostructures for in vivo administration having a discontinuous shell with divalent or trivalent iron ions coordinationally bonded to cross-linker-derived hydrophilic groups on the surface of a spherical core can be used as a cerebral cardiovascular system-specific contrast agent that can show a very strong and long-lasting contrast effect compared to current clinical GBCAs.

In general, conventional contrast agents are limited in the temporal scanning window because the contrast effect disappears within a short period of time, but the in vivo injectable nanostructures of the present invention have a long-lasting contrast effect and are less time-consuming, allowing the scanning time to be increased as needed to improve the spatial resolution of MRI. In addition, in clinical practice, when a patient moves during an MRI, image distortion often occurs and reshooting is required, and the nanostructures for in vivo administration of the present invention are effective for such reshooting due to their long-lasting contrast effect. In addition, if the MRI scan cannot be started immediately after the administration of the contrast agent due to various circumstances in the clinical field, the contrast effect is often lost and the examination is impossible, but the contrast effect is effective in such cases because it lasts for a long time.

Thus, the nanostructures for in vivo administration of the present invention may provide an opportunity to visualize clinically important microvessels in vivo with high spatial resolution via MRI (FIG. 4, Example 7). Furthermore, magnetic resonance imaging (MRI) can be used to determine tissue accumulation and/or extravasation in the body.

Further, from what can be determined by MRI imaging that nanostructures for in vivo administration that function as T1 MRI contrast agents according to the present invention are largely excreted into the urine via renal filtration after in vivo administration (FIGS. 6 and 7); and from what can be collected in urine after intra-articular injection (FIG. 12), it can be seen that in the route of administration, distribution and elimination in the body, the nanostructures for in vivo administration of the present invention are stable without leaching of divalent and trivalent iron ions coordinationally bound to hydrophilic groups on the surface of the spherical core in urine as well as in body fluids including plasma after in vivo administration.

On the other hand, gadolinium and manganese are elements that do not exist in the human body in their natural state, and when used as contrast agents, they remain in the body and cause side effects such as permanent deposits and skin sclerosis.

On the other hand, since iron is abundant in human blood and is the central atom of hemoglobin, an important molecule that binds oxygen in human red blood cells, and is one of the main elements that make up the human body, such that iron deficiency results in iron deficiency anemia, nanostructures for in vivo administration having a shell composed of divalent or trivalent iron ions according to the present invention are more biocompatible than gadolinium-based or manganese-based materials.

Superparamagnetic iron oxide nanoparticles (SPIOs) injected intra-articularly have been reported to remain in the joint without excretion for substantially longer periods of time.

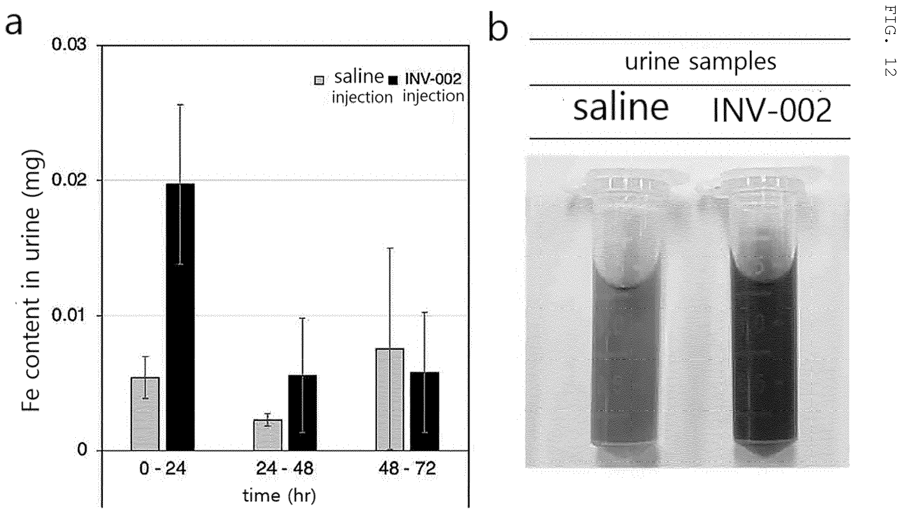

However, according to the present invention, nanostructures for intra-articular injection comprising a discontinuous shell with divalent to trivalent iron ions coordinationally bonded to cross-linker-derived hydrophilic groups on the surface of the spherical core are completely absorbed by the body and safely excreted from the body via the renal elimination pathway without any harmful effects on the joint tissue upon intra-articular injection (FIG. 12). In other words, the nanostructures for intra-articular injection with shells composed of divalent or trivalent iron ions according to the present invention are the first to be completely absorbed into the blood circulation after intra-articular injection without iron leaching, without accumulation in the joint cavity, and with complete renal elimination. Thus, the in vivo injectable nanostructures with shells composed of divalent or trivalent iron ions, when injected into the joint area, are absorbed into the blood circulation without being phagocytosed by macrophages, and are excreted through the kidneys into the urine without leaching iron from the tissues, making the side effect of iron accumulation, which is a cause of joint disease, extremely unlikely. All of these features suggest that the nanostructures for in vivo administration comprising a discontinuous shell with divalent or trivalent iron ions coordinationally bound to crosslinker-derived hydrophilic groups on the surface of a spherical core according to the present invention have remarkable potential as T1 MRI contrast agents for MR arthrography, as well as for use as MRI contrast agents, drug carriers, or adsorbents for collecting information in tissue or blood over a wide dose range without side effects and/or toxicity after in vivo administration.

Further, with respect to the various uses of nanostructures for in vivo administration having a shell comprising divalent or trivalent iron ions in accordance with the present invention, all of the contents of Korean Patent Application No. 10-2022-0028150 are incorporated herein by reference.

### [Overall size and surface charge design of nanostructures for in vivo administration].

After in vivo administration, whether it is phagocytosed by macrophages, whether it is metabolized, whether it is circulated in blood, whether it is circulated in lymph, whether it is delivered to the parenchyma of cells through capillaries, whether it is accumulated in tissues, whether it is excreted in urine through renal filtration, whether it is absorbed into the vascular circulation after in vivo administration, whether it is leaked through the blood vessel wall, and whether it can be collected and reused through urine can be realized by controlling the size of the nanostructure for in vivo administration according to the present invention.

Whether organic and inorganic nanoparticles exert an enhanced permeation and retention (EPR) effect, which means that when circulating in the body, they are more likely to selectively accumulate in cancer or disease cells with a loose structure than in normal tissues with a dense structure, can also be realized by controlling the size of the nanostructures for in vivo administration according to the present invention.

Thus, customized biocompatible nanostructures for in vivo administration that exert desired pharmacokinetic and pharmacodynamic properties can be designed by varying the molecular weight of the dextran monomer in the spherical core, the length of the dextran scaffold, the type of crosslinker at crosslinking, the amount and rate of crosslinker administered during the synthesis reaction, and/or additional chemical functional group modifications, all of which are related to the size of the nanostructure.

On the other hand, the surface charge of nanostructures for in vivo administration strongly influences the pharmacokinetics and behavior of intravenously injected nanomaterials. For example, when serum proteins non-specifically bind to nanomaterials via charge (i.e., opsonization), nanomaterial-protein complexes are formed, which promote their uptake by the mononuclear phagocyte system (MPS) and accumulation in organs. Even if the nanomaterials are naturally of a size that allows for renal filtration, their binding to proteins increases their size and makes them unavailable for renal excretion. To avoid this unintended long-term accumulation and produce nanoparticles that can be excreted via renal filtration, it is essential to charge the nanoparticles to effectively prevent opsonization.

Therefore, the nanostructures for in vivo administration according to the present invention can have a surface charge of -20 mV to 0 mV to control blood circulation time and renal clearance to avoid non-specific adsorption of serum proteins. Positively charged nanostructures are undesirable because they can cause nonspecific electrostatic binding to most negatively charged cells in vivo.

The desired surface charge can be designed by adjusting the mass ratio of spherical core dextran to iron from 100:2 to 100:10, adjusting the percentage of crosslinker substitution to 10% to 50% of the number of dextran functional groups, adjusting 20% to 50% of the crosslinkers so that one end is exposed without participating in crosslinking, and/or adjusting the percentage of crosslinker-derived hydrophilic groups so that only 20% to 80% of the exposed functional groups bind iron ions.

### [Size and colloidal stability of nanostructures for in vivo administration].

The nanostructures for in vivo administration of the present invention can improve colloidal stability in biological fluids through hydration of hydrophilic functional groups exposed on the surface of the dextran spherical core.

As used herein, biological fluids can be intracellular or extracellular. Extracellular fluids include blood, lymphatic fluid, and interstitial fluid that surrounds cells.

Nanoparticles with a hydrodynamic diameter of 1 to 30 nm can escape from phagocytes and travel through blood vessels. Typically, nanoparticles need to be 9 to 10 nm in size or smaller to be excreted naturally through the kidneys.

Therefore, the nanostructures for in vivo administration of the present invention can be designed to have an overall diameter of 10 nm or less, preferably 8 nm to 1 nm, more preferably 8 nm to 1.5 nm, and a uniform size distribution. If the diameter exceeds the above values, it is difficult to show uniform distribution and excretion in the body.

On the other hand, the size of the nanoparticle determines which organs they are ingested into and their subsequent distribution in the body. Nanoparticles larger than 50 nm are rapidly accumulated in the liver by the liver's Kupffer cells, and even nanoparticles with small cores can be easily aggregated if they are not dispersible, resulting in high uptake by the reticuloendothelial system.

The dextran-based core, which affects the overall size of the nanostructures for in vivo administration of the present invention, is two to three dextran molecular cross-links formed through intramolecular and intermolecular cross-links upon cross-linking of dextran by a crosslinker, thereby inhibiting water swelling, so that the core has a uniform size distribution. In addition, the nanostructures for in vivo administration of the present invention form a discontinuous shell composed of divalent to trivalent iron ions through coordination bonding with divalent to trivalent iron ions as ligands, as some of the hydrophilic groups exposed on the surface of the dextran-based spherical core, and the one hydrophilic groups of the crosslinker at the terminal end, connected to the dextran are exposed to the aqueous environment through the discontinuous exposed surface of the shell, so there is no aggregation of nanoparticles in vitro and in vivo, and the storage stability is excellent. As a result, it can be rapidly distributed in the blood and uniformly sized to produce a uniform contrast effect. In addition, imaging can be observed for more than 1 hour and up to 2 hours, and the hydration diameter can be maintained at a uniform size (10 nm or less), which reduces the uptake by the reticuloendothelial system in the liver and increases the residence time in the bloodstream, and it does not accumulate in the body and is excreted through the kidneys without metabolic degradation in the liver, solving the problem of conventional gadolinium-based contrast agents.

The nanostructures for in vivo administration of the present invention do not degrade in the body and maintain colloidal stability in body fluids through hydration, so they can be designed to be filtered from the blood vessels to the kidneys, collected in the bladder within an hour after injection, and largely excreted from the body in the urine. Therefore, they are not only recyclable, but can also be used to collect information in tissues or blood at the site of administration through adsorption.

### [Contrast Agent].

The nanostructures for in vivo administration of the present invention can be used as MRI contrast agents for MR angiography, MR arthrography, MR cisternography, MR myelography, MR lymphangiography, MR cholangiopancreatography, or brain and abdominal MRI imaging.

The nanostructures for in vivo administration of the present invention, which have been shown in animal studies to be able to be used as a positive contrast agent, are a type of drug that maximizes the signal of magnetic resonance imaging by reducing the proton relaxation time of the administered tissue water molecules, thus allowing the structure and function of biological tissues to be observed with higher contrast. The nanostructures for in vivo administration of the present invention can have a T1 relaxation rate (R₁ = 1/T₁) of 5 sec⁻¹ under physiological conditions (pH 7.4, 37 °C) and at 3.0 Tesla, which is the most common magnetic field strength of current MRI machines.

The relaxation rate of a contrast agent is directly affected by its concentration, with a proportional increase in signal up to a certain concentration range, after which the signal drops. For this reason, other contrast agents to date have required separate dilutions in clinical practice to achieve optimal concentrations. In contrast, the nanostructures for in vivo administration of the present invention exhibit high signal without dilution and can be prepared at optimal concentrations to facilitate observation and diagnosis of biological tissues.

Furthermore, the nanostructures for in vivo administration of the present invention can not only contrast with surrounding tissues (FIG. 10), but depending on the concentration distributed in the body fluid, the signal magnitude of the body fluid in the MRI varies, making it possible to determine the distribution in the tissue time series.

When the nanostructures for in vivo administration of the present invention are used as contrast agent drugs for in vivo administration, they can be used by linking them to drug carriers such as fragments, antibodies, aptamers, artificial antibodies (repebody), and the like that contain target antigen binding sites. In this case, it can be targeted to cells with target antigens on their surface or to target antigen-containing areas of the body, allowing imaging/quantification of the target antigen distribution/concentration at those areas by T1 MRI signal intensity, as well as enhanced efficacy such as high tumor uptake or rapid clearance from healthy tissue when using tumor antigen-specific antibodies.

Furthermore, the optimal position of the syringe needle for injecting the contrast agent during MR arthrography, MR encephalography or MR myelography is determined using fluoroscopy. The injection pattern of a small amount of iodinated X-ray contrast is monitored with fluoroscopic X-ray to ensure that the needle is in the correct position, after which the MRI contrast agent is injected. Therefore, the MRI contrast agent is inevitably mixed with the iodinated X-ray contrast, for example within the joint cavity. There are reports that the T1 MRI contrast effect of GBCA is lowered by iodinated contrast agents. The nanostructures for in vivo administration of the present invention exert a T1 contrast effect even after mixing with iodinated X-ray contrast agents, making them effective for clinical MR arthrography, MR hydroencephalography or MR spinal cord imaging.

Meanwhile, nanostructures for intra-articular injection comprising a discontinuous shell with divalent to trivalent iron ions coordinationally bonded to cross-linker-derived hydrophilic groups on the surface of a spherical core according to the present invention provide adequate soft tissue contrast upon intra-articular injection to clearly visualize the complex anatomy of the joint (FIG. 10) and show significantly greater and longer-lasting T1 MRI contrast effect compared to conventional gadolinium contrast agents.

### [Circulates in the blood and is excreted in the urine through the kidneys].

Typically, nanoparticles need to be 9-10 nm in size or smaller to be excreted naturally through the kidneys.

An important feature of the nanostructures for in vivo administration of the present invention, comprising a discontinuous shell with divalent or trivalent iron ions coordinationally bonded to crosslinker-derived hydrophilic groups on the surface of a crosslinked dextran-based spherical core, is that they exhibit excellent colloidal stability without aggregation and free iron leaching, thereby maintaining a compact hydrodynamic diameter smaller than the renal filtration size limit, resulting in excellent in vivo excretion. Unlike conventional nanoparticle contrast agents that circulate in blood vessels for unnecessarily long periods of time and often accumulate in organs, the nanostructures for in vivo administration of the present invention can be finely tuned to an average hydrodynamic size of less than 5 nm and exhibit colloidal stability in the body without aggregation, so that they can be completely excreted from the body through the kidneys without alternation.

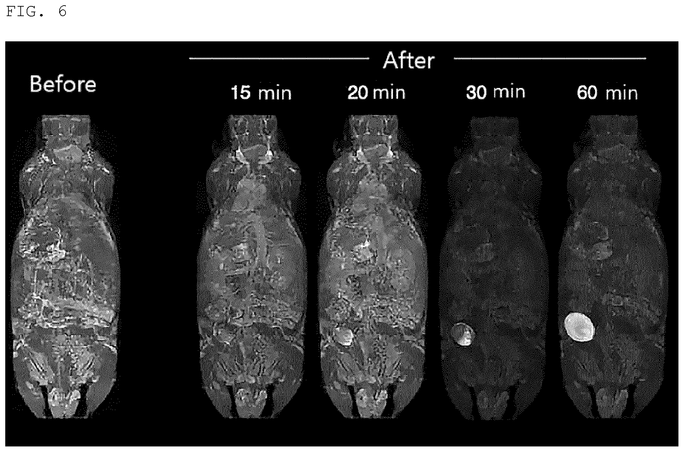

An MRI image of a rat obtained after intravenous injection of a nanostructure for in vivo administration of the present invention shows bright contrast in the bladder (FIG. 6). The contrast enhancement in the bladder shows that the nanostructures for in vivo administration of the present invention were injected into the blood vessels and then excreted unchanged by the kidneys through filtration and urination.

Furthermore, the nanostructures for in vivo administration of the present invention can be absorbed into the blood circulation after in vivo administration and excreted through the kidneys into the urine without extravasation through the blood vessel wall.

Therefore, the nanostructures for in vivo administration of the present invention can be excreted through the kidneys without alternation, thereby enabling information collection in the injected tissue, and can be used as MRI contrast agents to realize cardiovascular, cerebrovascular, lymphatic, musculoskeletal, and/or craniospinal nervous system imaging and/or quantification.

### [Phagocytosis by macrophages].

Drug nanoformulations hold the promise of selectively delivering drugs to the site of disease or delivering larger amounts of drug to the site of disease. Unfortunately, most drug nanoformulations, including artificial nanocarriers, liposomes, and polymeric nanoparticles, have limitations, such as being rapidly cleared from the blood circulation by the reticuloendothelial system (RES), one of the body's immune systems, before reaching the site of disease.

According to the present invention, nanostructures for in vivo administration comprising a discontinuous shell with divalent or trivalent iron ions coordinationally bonded to crosslinker-derived hydrophilic groups on the surface of a crosslinked dextran-based spherical core have structural features that can be modulated by various regulatory factors so that they are neither phagocytosed by macrophages nor bound by intravascular opsonin proteins. Thus, they can provide a desired blood circulation and renal clearance profile.

The reticuloendothelial system is also known as the macrophage system and the mononuclear phagocyte system (MPS). These are cells that absorb certain substances from different parts of the body. They form part of the body's defense mechanisms.

Reticuloendothelial cells are made from progenitor cells in the bone marrow. Progenitor cells develop into monocytes, phagocytic cells that are released into the bloodstream. Some monocytes remain in the circulation, but most enter body tissues and become much larger phagocytic cells called macrophages. The majority of macrophages remain in the tissue as immobile cells, filtering and destroying foreign particles. However, some break off and float around in the circulation or in the intercellular space.

Macrophages in tissues have different shapes and names depending on where they are located. Reticulocytes are found in lymph nodes, spleen, and bone marrow, while histiocytes are found in subcutaneous tissue. Microglia are found in nerve tissue, alveolar macrophages in the alveoli of the lungs, and Kupffer cells in the liver. A single reticuloendothelial cell can engulf microbes, cells, and even small pieces of foreign material such as bone fragments or sutures. Several motile macrophages can also fuse into a single phagocytic cell that surrounds a large foreign body. Through phagocytosis, macrophages form the first line of defense against harmful particles that enter the body.

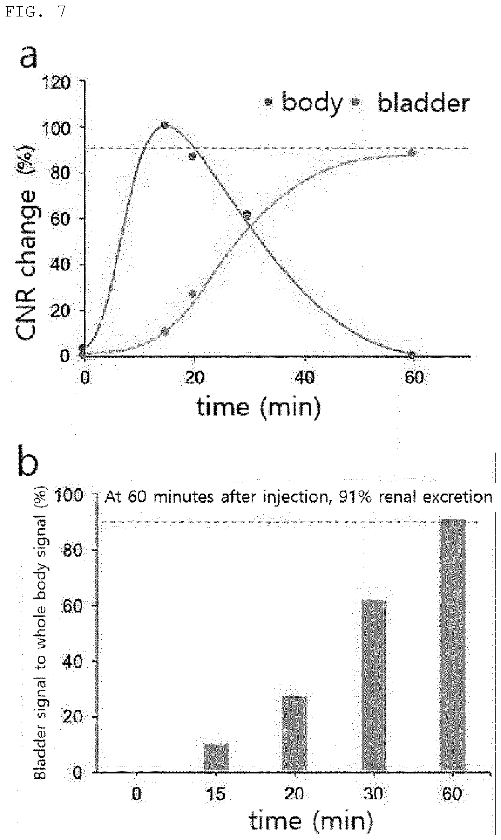

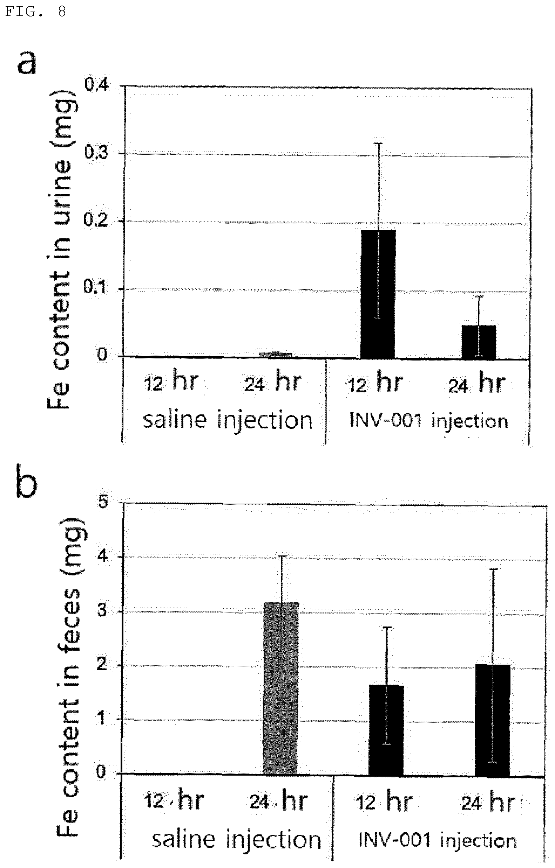

A nanostructure for in vivo administration that functions as a T1 MRI contrast agent according to the present invention enables the following to be noted: (FIGS. 6 and 7) that after in vivo injection, it can be seen via MRI that it is mostly excreted via renal filtration into urine; (FIG. 8) that after in vivo injection, it is mostly collected in urine rather than feces; and (FIG. 12) that after intra-articular injection, it can be collected in urine, indicating that the nanostructures for in vivo administration according to the present invention are not phagocytosed by macrophages and are not metabolically degraded along the route of administration, distribution and elimination in the body.

The nanostructures for in vivo administration of the present invention are not phagocytosed by macrophages along the route of administration, distribution, and elimination in the body after in vivo administration, and therefore are not absorbed by the liver, spleen, bone marrow, lymph nodes, etc. due to macrophage phagocytosis in organs of the trabecular endothelial system.

Furthermore, that the nanostructures for in vivo administration that function as T1 MRI contrast agents according to the present invention are not distributed in the liver, spleen, bone marrow, lymph nodes, etc. when administered intravenously or intra-articularly or intrathecally, can be confirmed by MRI imaging, and can be collected in urine without alteration.

Thus, nanostructures for in vivo administration comprising a discontinuous shell with divalent or trivalent iron ions coordinationally bonded to crosslinker-derived hydrophilic groups on the surface of a crosslinked dextran-based spherical core can be designed so that they are not phagocytosed by macrophages, and thus can be excreted intact into the urine without metabolic degradation along the pathway of absorption, distribution and/or excretion after in vivo administration, and can be collected in the urine for reuse or recycling.

Furthermore, the nanostructures for in vivo administration of the present invention can be designed to not be phagocytosed by macrophages, while at the same time having the function of a T1 MRI contrast agent that exhibits a bright signal in MRI images, so that the location of the nanostructures can be tracked via MRI images after in vivo injection to determine whether they are phagocytosed by macrophages after in vivo administration, metabolic degradation, blood circulation, lymphatic circulation, delivery to the cell's parenchyma through capillaries, accumulation in tissues, excretion in the urine through the kidneys, absorption in the vascular circulation after in vivo administration, leakage through blood vessel walls, and collection and reuse through urine.

### [In vivo Behavior Capillary Permeability]

The nanostructures for in vivo administration of the present invention may be designed so that, when administered intravenously, they can be eliminated from the blood circulation through renal filtration without leakage through the vessel wall (Example 1), or they may be designed so that, after injection into tissues other than blood vessels, such as joint cavities, spinal cavities, they can be absorbed into the blood circulation and eliminated from the blood circulation through renal filtration without leakage through the vessel wall (Example 2).

In general, drug distribution is the process by which a drug reversibly leaves the bloodstream and enters the extracellular fluid and tissues. Intrinsic factors such as cardiac output, local blood flow, capillary permeability, and tissue volume, as well as physicochemical properties of the drug such as size, relative lipophilicity, and binding of the drug to plasma/tissue proteins, are involved in the distribution of intravenously administered drugs. In this respect, the nanostructures for in vivo administration of the present invention can be designed to be capillary impermeable, such that when administered intravenously, they circulate in the blood and are excreted through the kidneys into the urine without leakage through the blood vessel wall or entry into the interstitial fluid (Example 1).

Capillary structure is highly variable in that it is a fraction of the basement membrane exposed by slit junctions between endothelial cells. In the liver and spleen, much of the basement membrane is exposed by large discontinuous capillaries, allowing passage of large plasma proteins. The capillary structure of the brain is continuous, with no gap junctions. Tightly parallel cells form tight junctions that make up the blood-brain barrier (BBB). For drugs to enter the brain, they must pass through the capillary endothelial cells of the central nervous system (CNS) or be actively transported.

The chemical nature of a drug strongly influences its ability to cross cell membranes. Fat-soluble drugs are soluble in lipid membranes, allowing them to pass through all cell faces. Hydrophilic drugs, on the other hand, do not easily cross the cell membrane and must pass through slit junctions.

The nanostructures for in vivo administration of the present invention are formed such that the hydrophilic functional group at one end of the crosslinker connected to the dextran can be exposed to the aqueous environment through the non-continuous exposed surface of the shell, and thus can be designed to be capillary impermeable, i.e., unable to pass through cell membranes as well as the endothelial intercellular slit junctions of capillaries, corresponding to hydrophilic drugs.

### [Absorbed and reused in the blood circulation after in vivo administration].

The circulatory system is responsible for the flow of fluids such as blood and lymphatic fluid that provides nutrients, oxygen, and energy to each organ in the body and carries carbon dioxide and waste products from life's activities to the respiratory and urinary systems for removal from the body.

The circulation of blood is driven by the work of the heart. Circulating blood transports oxygen, provides nutrients, removes waste products from metabolic processes, maintains body temperature, and carries hormones.

Significant amounts of ions, nutrients, organic wastes, dissolved gases, and water are permeable through capillaries, most of which are reabsorbed back into the capillaries. About the same amount of fluid leaves the capillaries and returns to the capillaries, and only a small portion is absorbed through the lymphatic vessels. From there, it flows into the lymphatic vessels and returns to the bloodstream.

In multicellular organisms, cells are morphologically and functionally differentiated and are generally arranged in groups of cells of the same type to perform a specific function. These organized cell populations are called tissues.

Animal tissues can be divided into epithelial, connective, cartilaginous, bone, blood and lymphatic, muscle, and nerve tissues based on their form and function.

Epithelial tissue is a tightly packed covering of one to several layers of cells that lines the surfaces of the body, the tubular cavities of the digestive and respiratory systems, and the peritoneal and pericardial cavities. Neighboring cells are closely packed and have little intercellular space. Epithelial tissues are sometimes embedded and form groups of secretory cells (glandular tissue), sensory epithelia of vision, hearing, and equilibrium, and epithelia with specialized properties such as hair and nails.

Muscle tissue is made up of muscle cells that perform contractile movements. Myocytes are called myofibrils because of their overall thin, elongated, fiber-like appearance.

Nervous tissue is made up of nerve cells (neurons) and glial cells (glia), which carry out the wired transmission of biological information. In higher animals, the brain and spinal cord are called the central nervous system, and the branches that branch off from it are called the peripheral nervous system. The central nervous system is made up of nerve tissue plus blood vessels and connective tissue. The peripheral nervous system is also composed primarily of nerve cells, fibers, and the Schwann cells (equivalent to glial cells) that surround the fibers.

The four tissues of connective tissue, cartilage, bone, and blood and lymph are collectively referred to as supportive tissues. Supportive tissues are rich in cellular interstitium, and bone, cartilage, and connective tissue help maintain the shape of the body and organs. The interstitium is made up of fibers and matrix, and cells are embedded and scattered within it. Blood and lymphatic fluids are embedded in supportive tissues because plasma and lymph are the matrix, and fibrin is the fiber.

A lymphatic vessel is a transparent, one-way, closed tube that transports liquids and substances from the lower layers of the skin or mucous membranes into the vascular system. Lymphatic vessels are small tubes that carry lymph from tissues to lymph nodes and then from the lymph nodes back to the blood vessels. Lymphatic vessels are more permeable than capillaries, allowing them to absorb macromolecules, including antigens and cells, more easily than capillaries. On the other hand, lymph nodes are distributed throughout the body along the lymphatic vessels and are where the lymphatic vessels and lymphatic channels connect.

Lymphatic fluid is a pale yellow fluid that contains less protein and more fat than blood, and contains more lymphocytes and white blood cells. Lymphatic fluid circulates throughout the body through the lymphatic vessels, delivering nutrients to each cell and taking away waste products. Lymphocytes are responsible for the immune response, defending the body against bacteria, viruses, and other infections.

As blood circulates throughout the body through the arteries and out of the veins, some fluid is left between cells, creating interstitial fluid (extracellular fluid that fills the spaces between tissues or cells where plasma has been filtered out of the capillaries in the tissue). When this interstitial fluid leaves the lymphatic capillaries, it is called lymphatic fluid. Lymphatic fluid flows at a very slow rate into the lymphatic vessels and finally re-enters the bloodstream. Lymphatic fluid flows out of the capillary walls and bathes the body's tissue cells, removing waste from the tissues.

As described above, this fluid circulates throughout the body through the lymphatic vessels, delivering nutrients to each cell and carrying carbon dioxide (CO₂) out of the cells and waste products such as damaged cells, cancer cells, and bacteria. After completing this process, the lymph reenters the bloodstream at the junction of the jugular and subclavian veins. As lymph flows through the lymph nodes, the lymphocytes in the lymph nodes react with foreign substances to remove them and destroy others. This is why lymph nodes swell when they filter out foreign substances, such as during acute inflammation.

Accordingly, the nanostructures for in vivo administration of the present invention can be injected into the periphery of the body and absorbed into the lymphatic vessels, or they can be designed for direct injection into the lymphatic vessels, in which case they can flow into the lymphatic vessels and be absorbed into the blood circulation without being eliminated by lymphocytes and macrophages in the lymph nodes.

In accordance with the present invention, nanostructures for in vivo administration comprising a discontinuous shell with divalent or trivalent iron ions coordinationally bonded to crosslinker-derived hydrophilic groups on the surface of a crosslinked dextran-based spherical core can be designed so that after administration into the body, they are completely absorbed into the blood circulation without accumulating in tissues and are collectible in the urine via renal filtration (FIGS. 6-8, FIGS. 10-14).

Thus, the nanostructures for in vivo administration of the present invention designed in this manner can be collected in the urine in an intact state without metabolic degradation along the pathway of absorption, distribution and/or excretion after in vivo administration, and thus can be reused or recycled. They can also be collected in the tissue, lymphatic system or blood at the site of administration, for example by adsorption.

### [Immune_response_toxicity].

As described above, the nanostructures for in vivo administration of the present invention can be absorbed into lymphatic vessels or injected directly into lymphatic vessels when injected into an extremity of the body, and can be absorbed into the blood circulation without causing acute inflammation that results in swollen lymph nodes because they are not removed by lymphocytes in the lymph nodes as the lymph flows through them.

In the blood, there are red and white blood cells, with white blood cells being the immune cells. White blood cells are made up of many different cells, including neutrophils, eosinophils, basophils, monocytes, platelets, and lymphocytes, and lymphocytes include B-lymphocytes, T-lymphocytes, and natural killer (NK) cells. Monocytes travel through blood vessels to tissues and differentiate into macrophages, which play a very important role in innate immunity. All of these white blood cells are involved in the immune response.

Innate immunity is a non-specific response to antigens that invade the body, regardless of the type, and does not have a specific memory.

The antigen is degraded in the macrophage, where it binds to major histocompatibility complex (MHC) molecules, and the bound fragments adhere to the cell surface. Through this process of antigen presentation, innate immunity (primary immunity) and adaptive immunity (secondary immunity) are linked, with innate immunity leading to secondary immunity.

In short, as described above, the nanostructures for in vivo administration of the present invention can be designed to avoid being phagocytosed by macrophages along the route of administration, distribution, and elimination in the body following in vivo injection, and thus do not act as antigens to induce immune responses related to innate and/or adaptive immunity, and thus do not cause diseases such as inflammation, allergy, hypersensitivity, abnormality, or syndrome.

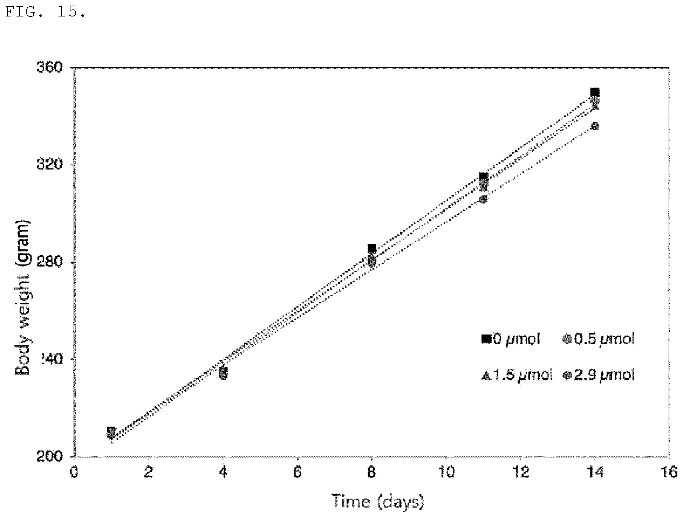

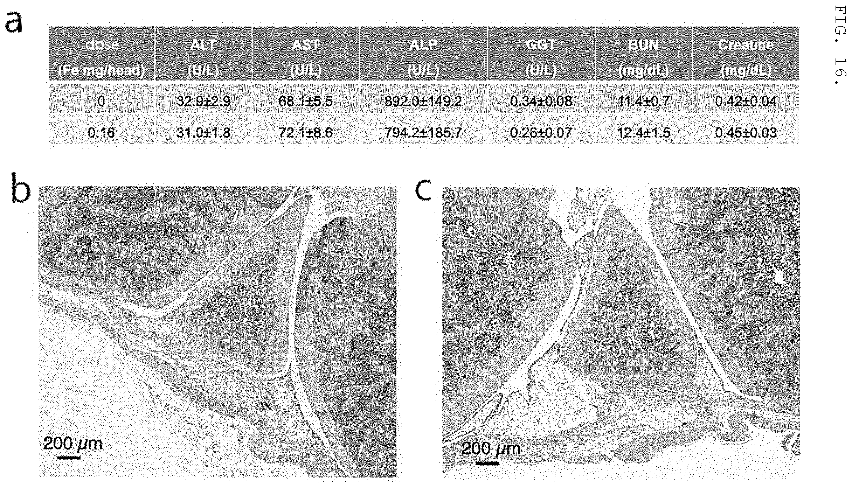

In animal experiments, the dextran-based spherical core crosslinked by a crosslinker according to the present invention and having a divalent or trivalent iron ion shell formed by coordination bonding of the cross-linking agent-derived hydrophilic groups exposed on the surface with a divalent or trivalent iron ion as a ligand is not degraded in the body, Even at high doses, it does not induce weight loss (FIG. 15), all blood chemistry tests showed normal ranges (FIG. 16), and no pathological abnormalities or lesions were observed in histopathology results (FIG. 16). In other words, the nanostructures of the present invention can be sufficiently designed to be non-toxic and exhibit excellent in vivo compatibility.

### [Intravenous administration_Nanostructures for blood circulation].

The nanostructures for in vivo administration of the present invention can circulate in the cerebral cardiovascular system. Furthermore, the nanostructures for in vivo administration of the present invention are not removed from the liver when injected into a vein, so they can be used as blood circulating nanostructures that are discharged into the urine through the kidneys after circulation. Therefore, it is possible to collect information in the blood while circulating, and it is possible to act as a contrast agent while circulating in at least one of the carotid artery, heart, aorta, inferior vena cava, and cerebral blood vessels, enabling imaging of the vascular structure and morphology of each tissue/organ, and analysis of blood flow and hemodynamic information.

Nanostructures for in vivo administration according to the present invention can be designed to act as a T1 MRI contrast agent after intravenous injection, without leakage through the blood vessel wall, without being phagocytosed by macrophages, and without being metabolized and excreted through the kidneys into the urine (FIGS. 4 to 8). In animal experiments, a prototype example of a nanostructure for in vivo administration according to the present invention was filtered from the blood vessels to the kidneys after circulation, collected in the bladder within 1 hour after injection, and excreted from the body in the urine.

Furthermore, the nanostructures for in vivo administration according to the present invention can realize the appropriate physicochemical properties that should be possessed by an intravenous formulation, such as osmotic pressure and viscosity (Example 1, FIG. 2). The smaller the average molecular weight of the dextran monomer, the higher the concentration of the nanostructures of the present invention can be prepared, so that the viscosity of the intravenous formulation can be finely controlled by adjusting the degree of dilution (Examples 1 to 5 and FIG. 2).

The nanostructures for in vivo administration of the present invention can be designed to improve the spatial resolution of MRI by lengthening the scan time during MRI because the contrast effect is maintained for a relatively long time, thus enabling higher resolution imaging of the whole body vasculature in vivo. Thus, the nanostructures for in vivo administration of the present invention are characterized by the ability to image microvessels that are clinically important but difficult to observe with conventional contrast agents in vivo via MRI. For example, when imaging of a rat brain was performed with a spatial resolution of 0.078 mm x 0.078 mm x 0.078 mm using the nanostructure INV-001 of Example 1, microscopic cerebral blood vessels about 0.078 mm thick could be clearly observed (FIG. 4(c)). Considering that the most widely used 3 Tesla MRI machines in clinical practice currently have a spatial resolution of approximately 1 mm, the 0.078 mm resolution achieved with this nanostructure INV-001 represents an improvement of approximately 13 times.

### [Joint cavity and intrathecal space administration].

The nanostructures for in vivo administration of the present invention are characterized in that they are absorbed into the body without accumulating at the injected site, and the absorbed nanostructures are excreted through renal clearance.

For example, a nanostructure for in vivo administration of the present invention may be designed to be absorbed into the blood circulation upon injection into an articular cavity or intrathecal space and excreted in the urine via the kidneys.

Nanostructure INV-002 of Example 2 is a particle with a hydrated diameter of ~5 nm with iron bound to the dextran crosslinker. Nanostructure INV-002 of Example 2 has a number average molecular weight of ~32 kDa, which is approximately half that of serum albumin, and a hydrodynamic diameter of ~5 nm, indicating that the size of nanostructure INV-002 of Example 2 meets the size criteria required for venous drainage and is optimal for intra-articular injection use.

An articulation is a joint between two or more bones, or a bone-to-bone connection, and is an important part of the body that allows for movement. Between the bones in a joint is a thin layer of hyaline cartilage called articular cartilage. There is a synovial membrane, a cartilaginous sac-like structure, and a synovial tissue composed of synoviocytes that secrete a sticky fluid called synovial fluid. The synovial fluid contains proteins, salts, and hyaluronic acid and nourishes the joint surfaces and smoothes the joint area. The synovial fluid also contains white blood cells and lymphocytes.

White blood cells (leukocytes) are millions in number and protect the body by resisting infection by engulfing foreign particles in the blood and tissues or by forming antibodies. Lymphocytes are divided into two types, B cells and T cells, both of which identify and bind to foreign substances (antigens) in the body. These two types of lymphocytes are found in tissues and organs.

Blood vessels do not directly nourish the articular cartilage because they only reach the point where the fibrous layer of the joint capsule meets the lubricating membrane.

The joint cavity is filled with synovial fluid, a viscous fluid that reduces friction between the articular cartilage of synovial joints during movement. Synovial fluid is known to contain a variety of molecules, including hyaluronic acid, proteins, and enzymes. The substances in synovial fluid can be eliminated by draining into lymphatic or venous vessels connected to the body's circulatory system.

Although there is no clear understanding of the exact size limit for drainage, it is known that the synovium is generally permeable to relatively small substances such as albumin (molecular weight: ~66.5 kDa) and IgG (molecular weight: ~150 kDa). Therefore, smaller size contrast agents (< ~7 nm) are preferred for homogeneous distribution, effective MR arthrography and excretion of the contrast agent, and the nanostructures of the present invention can be designed to simultaneously meet these conditions.

Intrathecal injection is a method of injecting drugs through a needle into the spinal canal (spinal cord; vertebral canal; axial spinal canal) and subarachnoid space. The spinal canal is a series of vertebral foramina located in each vertebra. The spinal canal contains and protects the spinal cord, meninges, blood vessels, and peripheral nerves. Intrathecal injections also reach the cerebrospinal fluid (CSF) between the arachnoid membrane and the dura mater.

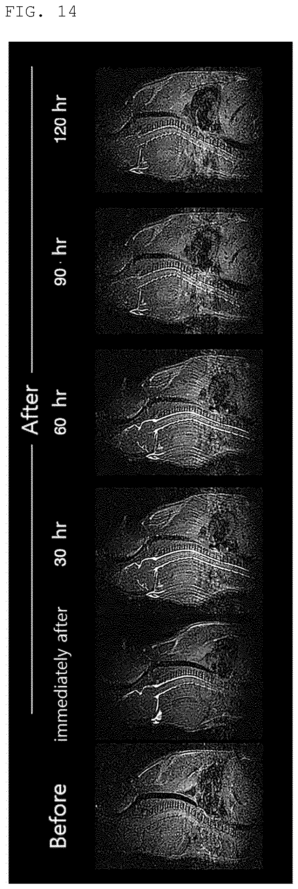

The nanostructures of the present invention can also be designed to allow for homogeneous distribution within the spinal canal/subarachnoid space, effective MR cisternography or MR myelography, and excretion of the contrast agent via intrathecal injection (Example 13, FIG. 14).

### [Lymphatic Administration_Nanostructures for Lymphatic Circulation] .

The nanostructures for in vivo administration of the present invention can be designed to be injected into a distal part of the body and absorbed into the lymphatic vessels or to circulate in the lymphatic fluid when injected into the lymphatic vessels.

The lymphatic system is an essential part of the immune system and includes organs such as the thymus, bone marrow, spleen, tonsils, appendix, and fire plates in the small intestine that produce and process specialized white blood cells that fight infection and cancer. The lymphatic system consists of (i) thin-walled lymphatic vessels, (ii) lymph nodes, and (iii) two collecting ducts.

Lymphatic vessels are distributed throughout the body, are larger than capillaries (the smallest blood vessels that connect arteries and veins), and are smaller than the smallest veins. Most lymphatic vessels have valves, like veins, to keep lymph flowing in one direction (toward the heart) that might otherwise get tangled. Tissues throughout the body drain a fluid called lymph, and lymphatic vessels carry lymph from tissues to lymph nodes and then return the fluid to the venous system through two collecting ducts.

Lymph begins as fluid that diffuses through the very thin walls of capillaries into the spaces between cells. Most of the fluid is reabsorbed by the capillaries, while the rest is drained into lymphatic vessels and ultimately returned to the veins. Lymph also contains many other substances, including (i) proteins, minerals, nutrients, and other substances that the fluid provides to tissues, and (ii) damaged cells, cancer cells, and foreign substances (such as bacteria and viruses) that enter the tissue fluid.

Lymph nodes are small, bean-shaped organs that function as collection centers for lymph. All lymph flows through strategically located lymph nodes to filter out damaged cells, cancer cells, and foreign bodies. Lymph nodes also contain white blood cells (such as lymphocytes and macrophages) that are specialized to phagocytize and destroy damaged cells, cancer cells, infectious microbes, and foreign bodies. Therefore, an important function of the lymphatic system is to remove damaged cells from the body and prevent the spread of infection and cancer. Some lymph nodes are clustered under the skin, especially in the neck, armpits, and groin. Other lymph nodes are located deep inside the body, for example, in the abdomen.

Lymph nodes drain into collecting ducts, which drain their contents into two subclavian veins located below the collarbone. These veins join to form the superior vena cava, which carries blood from the upper body to the heart.

The nanostructures for in vivo administration of the present invention may be designed to be injected into a peripheral site of the body and absorbed into the lymphatic vessels, or to be injected directly into the lymphatic vessels, in which case, when injected into a peripheral site of the body or directly into the lymphatic vessels, they may flow into the lymphatic vessels and be absorbed into the blood circulation without being eliminated by lymphocytes and macrophages in the lymph nodes.

Furthermore, if the in vivo injectable nanostructures of the present invention act as an MRI contrast agent, uptake/injection into the lymphatic vessels can enable MRI imaging of lymphatic vessel abnormalities such as lymphatic obstruction, lymphedema, enlarged lymph nodes, lymphadenitis, lymphoma, and migration of tumors from other organs to lymph nodes near the tumor.

### [Drug clearance Through Metabolism].

The nanostructures according to the invention are intended for in vivo administration and are therefore a type of drug.

Once a drug enters the body, the elimination process begins. The main routes of drug elimination are (1) hepatic metabolism, (2) biliary excretion, and (3) urinary excretion.

Elimination includes biotransformation (drug metabolism) and excretion. Excretion is the removal of the intact drug from the body.

A high percentage of nanoparticles remaining in organs such as the liver and spleen indicates unsatisfactory excretion performance.

It can be seen that the nanostructures for in vivo administration of the present invention can be eliminated from the body using (1) no hepatic metabolism, (2) no biliary excretion, and (3) only urinary routes of elimination, as evidenced by 100% collection in the urine after in vivo administration without accumulation in normal tissues.

Thus, the nanostructures for in vivo administration of the present invention can be collected via urinary excretion of at least 80%, preferably at least 90%, and even more preferably at least 95% of the injected dose.

### [Other pharmacokinetic properties].

Reversible binding to plasma proteins traps the drug in a non-diffusible form, delaying transport out of the vascular compartment. Albumin is a major drug binding site and acts as a drug reservoir. When the concentration of free drug in the blood decreases due to drug clearance, drug bound to albumin dissociates from albumin. Thus, the free drug concentration is maintained as a constant fraction of the total drug in the plasma.

However, the nanostructures for in vivo administration of the present invention, with a controlled charge of -20 mV to 0 mV, do not bind to plasma proteins such as albumin. This can be inferred from the fact that the nanostructures for in vivo administration of the present invention are filtered from the blood vessels to the kidneys, collected in the bladder and excreted from the body in the urine within one hour after injection.

Many drugs accumulate in tissue, resulting in higher drug concentrations in tissue than in interstitial fluid and blood. Drugs can accumulate due to binding to lipids, proteins, or nucleic acids. Drugs can also be actively transported into tissues. Tissue depots serve as a major resource for drugs and can prolong their action or cause localized pharmacotoxicity.

However, it can be inferred that the nanostructures for in vivo administration of the present invention are not bound to lipids, proteins or nucleic acids, as they are 100% collected in the urine after administration in the body without accumulation in normal tissues.

The nanostructures for in vivo administration of the present invention are completely eliminated from the body, including blood vessels, joint cavities, and spinal cavities, without accumulation or harmful effects, and can be safely removed from the body via the renal elimination route. Thus, the nanostructures for in vivo administration of the present invention can expand the potential for in vivo applications of nanomaterials through the successful demonstration of renal clearance pharmacokinetics after administration into the body (e.g., joint cavity, spinal cord cavity) as well as blood vessels.

Nanostructures for in vivo administration comprising a discontinuous shell with divalent or trivalent iron ions coordinationally bonded to crosslinker-derived hydrophilic groups on the surface of a crosslinked dextran-based spherical core are provided in accordance with the present invention, wherein the size of the nanostructures is controlled by the molecular weight of the dextran, the length of the dextran main chain, and the type of crosslinker at the time of crosslinking, By controlling the amount and rate of crosslinker administered during the synthesis reaction and at least one of the additional chemical functional group modifications, the overall size and total charge of the nanostructures can be controlled to impart desired blood circulation time and renal excretion pharmacokinetics.

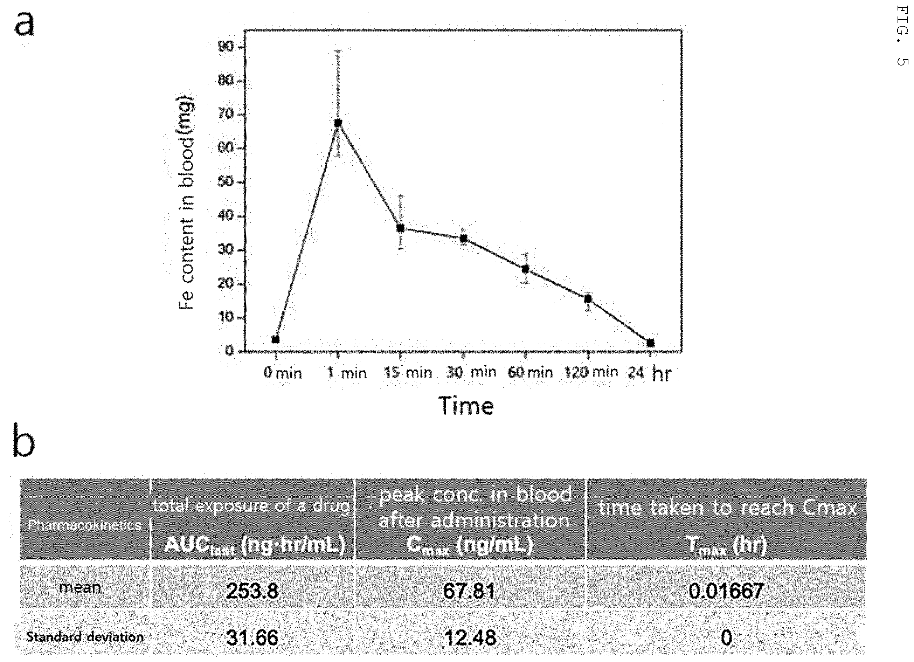

FIG. 5 exemplifies pharmacokinetic analysis data of a nanostructure for intravenous infusion (Example 1) according to one embodiment of the present invention. The nanostructures for in vivo administration according to the present invention exhibit a rapid half-life and can be dissipated after absorption into the vascular circulation.

### ADVANTAGEOUS EFFECTS

The present invention may provide nanostructures for in vivo administration that are not metabolized or degraded in the body after in vivo administration, are absorbed into the blood circulation, and are designed to be collected in the urine through renal filtration without leakage through the blood vessel wall for reuse, and can be used as reusable contrast agents and/or drug carriers or sorbents.

The nanostructures for in vivo administration of the present invention exhibit T1 MRI contrast effects similar to gadolinium-based contrast agents, distribute rapidly in the blood, allow vascular imaging for more than 1 hour, allow high-resolution imaging up to 0.078 mm x 0.078 mm x 0.078 mm, and can be excreted through the kidneys after contrast.

### DESCRIPTION OF THE DRAWINGS

FIG. 1 is a schematic of a nanostructure.
FIG. 2 shows a comparison of the concentration and viscosity at maximum concentration of the nanostructures for intravenous injection (codenamed INV-001) and intra-articular injection (codenamed INV-002).
FIG. 3 shows data on the T1 MRI contrast effect of the nanostructures.
(a-g) Comparative experimental data of T1 MRI contrast effect measurement of nanostructures INV-001 for intravenous injection. Sample photographs for T1 MRI contrast effect measurement (a), measured T1 MRI signal (b), measured *r*₁ (c) and *r*₂ values (d). (e) Comparison of *r*₂ /*r*₁ ratio of nanostructure INV-001 for intravenous injection and nanostructure INV-002 for intra-articular injection. (f) T1-weighted image (f) and T2-weighted image (g) of phantom containing INV-0002 solution of Example 2.
FIG. 4 shows data from an animal angiographic evaluation of INV-001, a nanostructure for intravenous injection. (a) T1 MRI images taken after intravenous administration of INV-001. (b) Time series graph of contrast-to-noise ratio (CNR) measured in the vascular system. (c) Three-dimensional MRI images taken at 0.078 mm x 0.078 mm x 0.078 mm spatial resolution after intravenous administration of INV-001 in rats, showing the rat brain in the sagittal, coronal, and transverse directions, respectively.
FIG. 5 shows the pharmacokinetic analysis data of the nanostructure INV-001 for intravenous injection. (a) Analysis of iron content in blood as a function of time after intravenous injection of INV-001. (b) Pharmacokinetic parameters of INV-001.
FIG. 6 shows T1 MRI image data taken at different times after intravenous injection of INV-001 in rat.
FIG. 7 shows the quantitative analysis data of body distribution and renal excretion of intravenously injected INV-001. (a) CNR changes in whole body and bladder after administration of INV-001. (b) CNR change in bladder compared to whole body CNR change.
FIG. 8 shows comparative urinary and fecal iron content data for INV-001 treated and saline treated controls. Comparison of (a) iron content in urine and (b) iron content in feces.

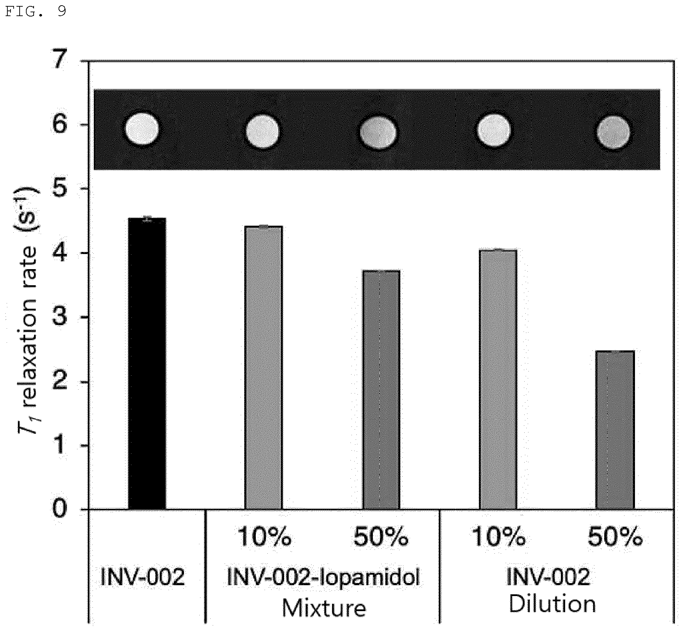

FIG. 9 is data showing the T1 MRI contrast effect of INV-002 when mixed with an iodinated X-ray contrast agent.
FIG. 10 shows rat magnetic resonance imaging arthrography efficacy data obtained by injecting INV-002 into a rat joint cavity. (a, b) Schematic representation of tissue structure in the joint cavity. (c, d) MRI images of rat joints of INV-002-injected experimental group and saline-injected control group. Comparison of (e) T1 MRI signal intensity and (f) CNR of intra-articular tissue before and after INV-002 injection.

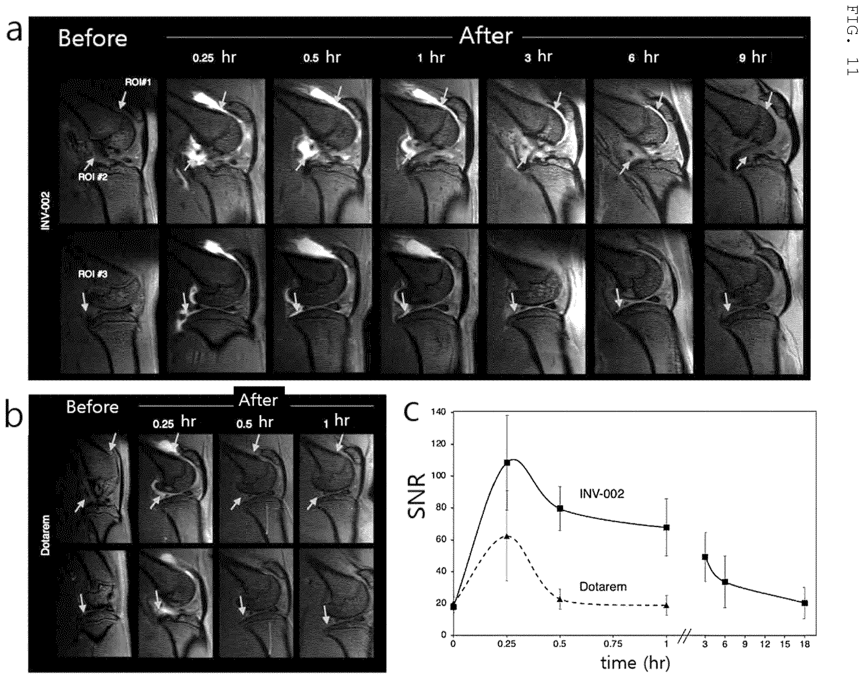

FIG. 11 shows data comparing the effectiveness of INV-002 and Dotarem in magnetic resonance imaging arthrography. Time series graphs of T1 MRI images after intraarticular injection of INV-002 (a) and Dotarem (b), and signal-to-noise ratio (SNR,c) measured at the joint cavity of a rat knee at different times.
FIG. 12 shows data comparing (a) the amount of iron in urine collected after intra-articular injection of INV-002 in rats and (b) the color of urine samples collected after saline injection.

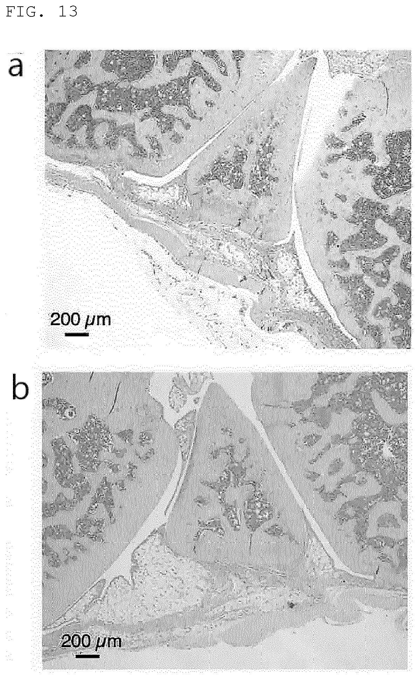

FIG. 13 is data from the analysis of trace iron in rat joint cavities using Perls Prussian blue tissue immunochemical staining. Photomicrographs of (a) INV-002-injected rat joint cavity tissue and (b) saline-injected rat joint cavity tissue.
FIG. 14 is a time series of T1 MRI images acquired after intrathecal injection of INV-002 in a rat spinal cord.
FIG. 15 shows weight pattern data measured over 14 days following intra-articular injection of INV-002 in rats.
FIG. 16 shows blood chemistry and histopathology data analyzed after intra-articular injection of INV-002 in rats. (a) Blood chemistry analysis results of INV-002 and saline injected into the joint cavity of rats. (b) Histopathology pictures of INV-002-injected experimental group and (c) saline-injected control group.

### Mode for carrying out the invention

Hereinafter, the present invention will be described in more detail through embodiments. However, the following embodiments are intended to clearly illustrate the technical features of the present invention and do not limit the scope of protection of the present invention.

### Example 1: Synthesis of nanostructures for intravenous infusion

180 µmol of dextran (average molecular weight 5,000 Da) was dissolved in 9 mL of distilled water, followed by 75 mmol of epichlorohydrin and 75 mmol of NaOH. Then, 380 mmol diethylenetriamine was added and stirred at room temperature (RT) for 24 hours. After 24 h of succinylation reaction at room temperature, the dextran core was purified by ultrafiltration with a 5 kD molecular weight cut-off (MWCO) filter. An excess of ferric chloride hexahydrate solution was added to the dextran core solution. The pH was adjusted to 8 using 1 M NaOH and purified by ultrafiltration after a 1 h reaction at room temperature to synthesize the nanostructure shown in FIG. 1 (code name: INV-001).

### Example 2: Synthesis of nanostructures for intra-articular injection

180 µmol of dextran (average molecular weight 10,000 Da) was dissolved in 9 mL of distilled water, followed by 75 mmol of epichlorohydrin and 75 mmol of NaOH. Then, 380 mmol ethylenediamine was added and stirred at room temperature (RT) for 24 hours. After 24 h of succinylation reaction at room temperature, the dextran core was purified by ultrafiltration with a 15 kD molecular weight cut-off (MWCO) filter. An excess of ferric chloride hexahydrate solution was added to the dextran core solution. The pH was adjusted to 8 using 1 M NaOH, and after a 1 h reaction at room temperature, the nanostructures (code name: INV-002) were purified by ultrafiltration.

### Example 3: Analysis of nanostructures

The nanostructures synthesized in Examples 1 and 2 above are yellow to yellowish brown transparent solutions when observed with the naked eye, and the content of iron and the content of dextran were analyzed by inductively coupled plasma (ICP) and phenol-sulfuric acid method, respectively, and the ratio was about 3 : 100 on a mass basis. The number of crosslinker-derived functional groups was determined by o-phthalaldehyde assay and elemental analysis after the crosslinking reaction, and it was analyzed that the crosslinker substitution ratio was 10% to 50% of the number of dextran functional groups, of which 20% to 50% did not participate in the crosslinking and the terminals were exposed to the outside. The crosslinker-derived functional groups were also detected when the phthalaldehyde quantification was performed again after binding iron ions, indicating that some of the crosslinker-derived functional groups are bound to iron ions and the remaining ones are exposed in an unbound form. The synthesized nanostructures were analyzed by dynamic light scattering and the hydrodynamic diameter was found to exhibit a uniform size distribution within the renal filtration cutoff of 8 nm and the charge was found to be -20 mV to 0 mV. The average molecular weight measured by gel permeation chromatography was approximately 10 ~ 15 kD for INV-001 and approximately 30 ~ 35 kD for INV-002, indicating that the nanostructures were synthesized from dextran with an average molecular weight of approximately 5,000 Da and 10,000 Da, respectively, and analyzed to be cross-linked by two or three dextran molecules.

### Comparative Example 1: 3 nm Iron Oxide Synthesis

The 3 nm iron oxide particles were synthesized according to a previous report. Briefly, 10 mmol of iron-oleate complex, 60 mmol of oleyl alcohol and 294 mmol of diphenyl ether were added to a 250 mL round bottom flask. The mixture was heated to 200 °C at a constant ramping rate of 10 °C/min and then cooled to room temperature. The synthesized nanoparticles were precipitated with excess acetone and then redispersed in tetrahydrofuran. 4 mL of iron oxide nanoparticles-tetrahydrofuran solution (10 mg/mL) was mixed with 400 mg of PEG-phosphate in ethanol and heated at 70°C for 8 hours. The final product was purified by a dynamic dialysis unit using 20 kDa MWCO.

### Example 4: Enrichment comparison of nanostructures

INV-001 of Example 1 and INV-002 of Example 2 were loaded into a centrifugal filter (Amicon Ultra-0.5 Centrifugal Filter, Millipore) with a 3 kDa molecular weight cutoff (MWCO) and concentrated using a fixed angle rotor centrifuge at 14,000 X g for 1 hour at 25°C with gravity acceleration. The iron concentration of the concentrated samples was quantified using inductively coupled plasma (ICP), and as shown in FIG. 2, INV-001 was analyzed to be approximately 2.6 mg/ml and INV-002 was analyzed to be approximately 1.6 mg/ml, indicating that INV-001 can be prepared at approximately 160% higher concentration than INV-002.

### Example 5: Comparing viscosity measurements of nanostructures

After diluting INV-001 to have the same concentration as INV-002, which was maximally concentrated in Example 4, INV-001 and INV-002 were placed in a test tube, and the test tube was placed in a constant temperature bath at 40°C for about 30 minutes before being removed and placed in a glass sample cup of a vibrational viscometer (SV-10, AND). The temperature of the sample gradually decreased over time and the viscosity was measured when it reached about 37.5°C. As shown in FIG. 2, the viscosity of INV-001 was analyzed to be about 25% lower than that of INV-002.

As can be seen from Examples 1 to 4 and FIG. 2, the use of dextran molecules with smaller average molecular weight allows the nanostructures of the present invention to be prepared in high concentrations, and thus the viscosity of the formulation can be finely controlled by adjusting the degree of dilution.

### Example 6: Comparison of T1 MRI contrast effect measurements of nanostructures

To analyze whether the above-described nanostructures have a suitable contrast effect as a T1 MRI contrast agent, the spin-spin relaxivity coefficient (*r*₂ ) and the spin-lattice relaxivity coefficient (*r*₁ ) were measured respectively, and the ratio (*r*₂ /*r*₁ ratio) was calculated. The *r*₂ /*r*₁ ratio is a measure of whether a contrast agent is suitable as a T1 MRI contrast agent or a T2 MRI contrast agent, and a typical T1 MRI contrast agent has a *r*₂ /*r*₁ ratio of about 1 to 2, and a T2 MRI contrast agent preferably has a *r*₂ /*r*₁ ratio of 5 or more.

As shown in FIG. 3, a contrast effect analysis of a 3.0 Tesla MRI phantom of INV-001 (a, b) in Example 1 determined that r₁ was 2.62 mM⁻¹ s⁻¹ (d) and r₂ was 2.73 mM⁻¹ s⁻¹ (c). The final calculated *r*₂ /*r*₁ ratio of INV-001 was 1.06, and INV-002 had a *r*₂ /*r*₁ ratio of 1.3 (d), indicating that both INV-001 and INV-002 have suitable contrast effects as T1 MRI contrast agents.

Also, as can be seen from (a) and (b) in FIG. 3, the INV-0001 sample with higher concentration shows a brighter signal.

Meanwhile, the *r*₁ and *r*₂ values of INV-0002 were measured in a 9.4 Tesla MRI scanner. FIG. 3 (f) is a T1-weighted image of a phantom containing the INV-0002 solution of Example 2. As can be seen in FIG. 3(f), the higher concentration sample shows a brighter signal. In contrast, no significant difference was found in the T2 highlight image at the higher concentration (FIG. 3(g)) .

In other words, the T1 MRI signal intensity of the nanostructures for in vivo administration of the present invention is a concentration dependence of the contrast agent, i.e., the higher the concentration, the stronger the T1 MRI signal intensity.

### Example 7: Evaluation of animal angiographic efficacy of nanostructured INV-001 for intravenous infusion

Angiography was performed with three-dimensional contrastenhanced T1 MRI-enhanced imaging on an animal MRI machine (Bruker BioSpin 9.4 Tesla). After a single intravenous administration of INV-001 of Example 1 to male Balb/c mice aged 7 weeks or older, the contrast-to-noise ratio (CNR) was evaluated and compared to a conventional gadolinium contrast agent (Dotarem). In addition, the duration of the contrast effect was determined and compared to a conventional gadolinium contrast agent.

As shown in FIG. 4(a), T1 MRI angiography after INV-001 injection in Example 1 revealed a bright cerebrovascular system compared to pre-injection, and the carotid artery, heart, aorta, and inferior vena cava of the mouse could be clearly observed.

As shown in FIG. 4(b), the CNR measured in the cerebrovascular system at first-pass was 4.87, representing a 200% improvement over gadolinium contrast. At steady-state, the CNR measured in the CNS was found to be 250% better than Dotarem, confirming that the contrast effect of INV-001 is stronger and longer lasting than gadolinium contrast agents.

The strong and long-lasting contrast effect of INV-001 of Example 1 was utilized to enable high-resolution MRI imaging. As shown in FIG. 4(c), the rat brain vessels were imaged with a spatial resolution of 0.078 mm x 0.078 mm x 0.078 mm, and microvessels as small as 0.078 mm could be clearly observed.

### Example 8: Absorption of nanostructured INV-001 for intravenous infusion

Blood absorption was evaluated following a single intravenous dose of INV-001 of Example 1 in male Sprague-Dawley rats 7 weeks of age or older. Blood was collected before INV-001 administration and at 1, 15, 30, 60, 120, and 24 hours post-dose, centrifuged to separate plasma, and iron concentrations in plasma were quantified using ICP.

As shown in FIG. 5(a), the pharmacokinetic absorption assay showed that the amount of iron in plasma as a function of time increased within 1 minute of INV-001 injection into a subcutaneous vein and decreased over time. By 24 hours after INV-001 injection, plasma iron levels had returned to pre-injection levels. As shown in FIG. 5(b), the AUCₗₐₛₜ for INV-001 was 253.8 ± 31.66 ng-hr/mL, Cₘₐₓ was 67.81 ± 12.48 ng/mL, and Tₘₐₓ was 0.01667 ± 0.00 hr, indicating that INV-001 has a rapid half-life and disappears after absorption in the body.

### Example 9: Excretion of nanostructures INV-001 for intravenous injection

Based on the results of Example 8, intravenously administered INV-001 was analyzed to be rapidly absorbed, and continuous MRI imaging was performed for 60 minutes to evaluate the distribution and excretion of absorbed INV-001. As a result, as shown in FIG. 6, a signal of INV-001 was observed in the bladder as early as 20 minutes after injection, and the signal became increasingly intense over time, with most of the signal appearing in the bladder at 60 minutes after injection. Analysis of the percentage of total MRI signal in the bladder showed that approximately 91% of the INV-001 in Example 1 was transported to the bladder within 60 minutes of injection, as shown in FIG. 7.

Following the above MRI analysis, urine and feces were analyzed for iron content to provide a more accurate analysis of excretion. Male Sprague-Dawley rats aged 7 weeks or older were administered a single intravenous dose of INV-001 of Example 1, and urine and feces were collected at time points ranging from immediately after dosing to 12 hours after dosing, and from 12 hours after dosing to 24 hours after dosing. Statistical significance was calculated for urinary and fecal iron content using non-parametric and parametric statistical methods, respectively.

As a result, as shown in FIG. 8(a), there was a statistically significant difference (p-value = 0.01996) between the iron content in urine collected from the INV-001-injected experimental group and the iron content in urine from the control group injected with nothing, confirming that INV-001 is excreted in urine. On the other hand, for feces, as shown in FIG. 8(b), no statistical significance was found in the iron content of the INV-001-injected experimental group and the non-injected control group.

Based on the above results, INV-001 of Example 1 was analyzed to be eliminated via urine after administration.

### Example 10: T1 MRI contrast effect of nanostructure INV-002 for intra-articular injection

To determine whether INV-002 of Example 2 can be used as a T1 MRI contrast agent for magnetic resonance imaging arthrography (MR arthrography), we tested whether the T1 MRI contrast effect was maintained after mixing with an iodinated X-ray contrast agent.

For this test, INV-002 is mixed with an iodinated X-ray contrast agent for the following reasons. In actual clinical practice, when performing magnetic resonance imaging arthrography, X-ray fluoroscopy is utilized to inject the contrast agent into the correct location within the joint cavity. To confirm that the syringe needle is in the correct position in the joint cavity, a small amount of iodinated X-ray contrast agent is injected and the pattern of the contrast spreading in the joint cavity is observed with a fluoroscopic X-ray. Once it is confirmed that the syringe needle is in the intended position, the MRI contrast is injected. Because of this, the MRI contrast inevitably mixes with the iodinated X-ray contrast agent within the joint cavity. It is known that some MRI contrast agents have been shown to decrease T1 MRI contrast when mixed with iodinated X-ray contrast agent.

For testing, a mixture of INV-002 of Example 2 and Iopamidol, one of the conventional iodinated X-ray contrast agents, was prepared. As shown in FIG. 9, the INV-002-Iopamidol mixture generally produced stronger T1 MRI contrast than the same concentration of INV-002. For example, a 1:1 mixture of INV-002 and Iopamidol produced approximately 2-fold stronger T1 MRI contrast than the same concentration of INV-002. The above results confirm that INV-002 of Example 2 can be effectively utilized for magnetic resonance imaging arthrography by maintaining a strong T1 MRI contrast effect even when mixed with an iodinated X-ray contrast agent.

### Example 11: Magnetic resonance imaging arthrography utilizing nanostructures INV-002 for intra-articular injection

Male Sprague-Dawley rats aged 7 weeks or older were anesthetized with a mixture of oxygen and isoflurane, INV-002 of Example 2 was injected into the knee joint, and T1-enhanced MRI images were acquired using a fast-spin echo sequence.

As shown in FIG. 10 (a-d), the knee joint cavity of INV-002-injected animals appears significantly brighter in MRI images compared to the non-injected control knee joint cavity. This change in signal intensity can also be confirmed quantitatively as shown in FIG. 10 (e). For example, the T1 signal intensity of the synovial fluid is enhanced from 4,900 (pre-injection) to 21,000 (post-injection). Next, the CNR of different tissues in the joint cavity were analyzed, including meniscus, capsule, cruciate, bone, and fatty areas (f). CNR is the difference between the average signal intensity of the synovial fluid and each anatomy divided by the standard deviation of the background signal intensity. After INV-002 injection, the CNR of the meniscus, capsule, cruciate, bone, and fat regions increased 10, 8.5, 18, 14, 12, and 4-fold, respectively, compared to pre-injection.

As shown in FIG. 11, the contrast effect of INV-002 was not only stronger than gadolinium contrast agents (e.g., Dotarem), but also lasted longer (a, b). For quantitative analysis of contrast effect, SNR was measured at two locations within the joint cavity (orange arrows) and the average value was plotted over time. INV-002 and Dotarem showed the highest SNR at 0.25 hours post-injection, with INV-002's SNR analyzed to be approximately twice as high as Dotarem (c). Dotarem's SNR decreased to pre-injection levels within 0.5 hours, while INV-002 maintained significant SNR until 6 hours post-injection, with no contrast enhancement observed from 9 hours post-injection.

From these results, it was analyzed that INV-002 in Example 2 showed intra-articular contrast enhancement, which may be helpful in the identification of intra-articular anatomical structures, and that the contrast effect lasted longer than that of Dotarem, which may provide useful opportunities for high-resolution imaging, repeat imaging, and retake imaging in case of failure.

### Example 12: Excretion of nanostructures INV-002 for intra-articular injection

Based on the lack of contrast enhancement observed at 9 hours after intra-articular injection of INV-002 into the rat knee, as seen in Example 11, it was determined that INV-002 was absorbed and cleared from the joint cavity within 9 hours. To analyze the elimination pathway more precisely, urine was collected after intra-articular injection of INV-002 into the knee joint cavity of Sprague-Dawley rats over 7 weeks of age. Urine collected at 0 to 24, 24 to 48, and 48 to 72 hours was analyzed for iron content using ICP. Urine was collected and analyzed for iron content at the same time points and methods after saline infusion in the control group.

As a result, as shown in FIG. 12(a), it was determined that the iron content in urine collected from INV-002-injected male and female rats was statistically significantly different from that of the saline-injected control group. In particular, the total amount of iron in the urine was analyzed to be approximately 0.018 mg, which is the same as the amount of injected iron within the margin of error, indicating that intra-articularly injected INV-002 can be excreted from the body within 24 hours of injection, mostly through urinary excretion. As shown in FIG. 12(b), the color of the urine sample collected after INV-002 injection was analyzed to be a darker brown color compared to the saline infusion control due to the color of INV-002.

To confirm that the INV-002 injected into the joint cavity was completely cleared, we used Perl's Prussian blue iron staining, which can detect even trace amounts of iron, and performed microscopic analysis. Joint tissues from INV-002-injected animals were collected, fixed in 10% neutral buffered formalin, subjected to Peris Prussian blue iron staining, and observed under a digital microscope. As shown in FIG. 13, both the INV-002-injected group (a) and the saline-injected control group (b) showed no iron accumulation.

From the above results, it was analyzed that INV-002 injected intra-articularly is excreted in the urine without intra-articular accumulation.

### Example 13: Contrast effect and Excretion of nanostructures INV-002 injected into the spinal cord cavity

Having confirmed that INV-002 injected into the articular cavity can be expelled from the articular cavity, as shown in Example 11, experiments were conducted to determine if INV-002 could also produce a contrast effect and be expelled when injected into the spinal cord cavity. Sprague-Dawley rats over 7 weeks old were anesthetized with a mixture of oxygen and isoflurane, INV-002 of Example 2 was injected into the spinal cord, and T1-weighted images were acquired using a fast-spin echo sequence.

As shown in FIG. 14, the spinal cord cavity of INV-002-injected animals was observed as a bright signal on T1 MRI images. Compared to pre-injection, the spinal cord cavity was brighter immediately after injection, with peak brightness at 30 and 60 minutes post-injection. The brightness decreased by 90 minutes post-injection and was similar to pre-injection by 120 minutes post-injection.

From the above, it was analyzed that the nanostructure INV-002 injected into the spinal cord cavity was discharged outside the spinal cord cavity.

### Example 14: Single-dose safety study of nanostructured INV-002 for intra-articular injection

To evaluate the intra-articular local toxicity of INV-002, INV-002 was administered as a single intra-articular dose to Sprague-Dawley rats and Beagle Dogs and observed for 2 weeks. The concentration of INV-002 used in the local toxicity test was 1.6 Fe mg/mL, the highest concentration available in Example 3, which is approximately 11 times higher than the concentration of the drug product intended for clinical use of 0.14 Fe mg/mL, and was administered in the maximum volume available for intra-articular administration in the test animals (0.1 mL in rats and 1 mL in Beagle dogs). In this case, the amount injected was 0.16 Fe mg/head in rats and 1.6 Fe mg/head in Beagle dogs.

As a result, no adverse events were observed, and as shown in FIG. 15, no differences in body weight patterns were observed between the INV-002-injected group and the no-injection control group. Therefore, the No Observed Adverse Effect Level (NOAEL) of 0.160 Fe mg/head and 1.60 Fe mg/head, respectively, were analyzed as 23 and 6 times the intended clinical dose, respectively, indicating excellent safety.

### Example 15: Single-dose hematology and histopathology testing of nanostructured INV-002 for intra-articular injection

Since INV-002 in Example 2 was found to be absorbed into the circulation and excreted via the renal elimination pathway, blood chemistry tests were performed to assess liver function (alanine transaminase, ALT; aspartate transaminase, AST; alkaline phosphatase, ALP; gamma-glutamyltransferase, GGT) and kidney function (blood urea nitrogen, BUN; creatinine, CR). Blood was drawn from the abdominal aorta for blood chemistry analysis and serum was separated by centrifugation. ALT, AST, ALP, GGT, BUN, and CR levels were analyzed using a biochemical automated analyzer.

For histopathologic examination, each organ was fixed in 10% neutral buffered formalin and processed for hematoxylin and eosin staining according to the protocol provided by the manufacturer. Tissue sections were counterstained with eosin for 1 minute and then analyzed by digital microscopy.

As a result, all blood chemistry tests were within the normal range as shown in FIG. 16, and histopathology results showed no pathological abnormalities or lesions, indicating that INV-002 has excellent in vivo compatibility.

No abnormal changes in cell structure (e.g., collapse, distortion, or expansion) were observed.

None of the organs showed bleeding, inflammation or necrosis, indicating the non-toxicity of INV-002. Blood chemistry analysis confirmed normal kidney and liver function in mice injected with INV-002.

## Claims

1. A nanostructure for in vivo administration that, after in vivo administration, is excreted in the urine via the kidneys without being phagocytosed by macrophages and/or metabolized, comprising
(i) a spherical core formed by crosslinking two or three dextran molecules with an average molecular weight of 10,000 Da or less using a crosslinker and (ii) a discontinuous shell with divalent or trivalent iron ions coordinationally bonded to crosslinker-derived hydrophilic groups on the surface of the spherical core; and
(iii) a mass ratio of dextran to iron ranging from 100 : 2 to 100 : 10, with a crosslinker substitution ratio adjusted to 10% to 50% of the total number of functional groups on the dextran, while 20% to 50% of the crosslinker does not participate in crosslinking at the terminal end, resulting in the nanostructure having the charge ranging from -20 mV to 0 mV;
(iv) wherein only 20% to 80% of the crosslinker-derived hydrophilic groups externally exposed on the surface of the spherical core bind iron ions, while the remainder do not bind iron ions and are exposed to an aqueous environment.

2. The nanostructure for in vivo administration of claim 1, wherein the divalent or trivalent iron ions coordinationally bonded to crosslinker-derived hydrophilic groups on the surface of the dextran spherical core are (a) stable without aggregation or free iron leaching in buffer solutions and plasma at physiological pH, and/or (b) capable of functioning as a T1 MRI contrast agent.

3. The nanostructure for in vivo administration of claim 1, wherein the T1 MRI signal intensity exhibited by the nanostructure is proportional to the concentration of the nanostructure, such that the concentration of the nanostructure is quantifiable from the signal of the magnetic resonance imaging (MRI) and/or the in vivo distribution of the nanostructure over time can be imaged or quantified.

4. The nanostructure for in vivo administration of claim 1, which is designed to function as a T1 MRI contrast agent that exhibits a bright signal in magnetic resonance imaging (MRI),
wherein (i) the location of the nanostructures can be tracked via MRI after in vivo administration and/or,
(ii) they can provide information about the pathways by which they are absorbed, distributed, metabolized, and/or excreted after in vivo administration, and/or the various anatomical structures and/or functions located along those pathways.

5. The nanostructure for in vivo administration of claim 1, which is designed to function as a T1 MRI contrast agent that exhibits a bright signal in magnetic resonance imaging (MRI),
wherein, at least one of the following can be determined: whether it is phagocytosed by macrophages after in vivo administration, whether it is metabolized, whether it is circulated in the blood, whether it is circulated in the lymph, whether it is delivered to the parenchyma of a cell via capillaries, whether it is accumulated in tissue, whether it is excreted in the urine via the kidneys, whether it is absorbed into the vascular circulation after in vivo administration, whether it leaks through the blood vessel wall, and whether it can be collected and reused via urine,
thereby providing a personalized biocompatible nanostructure that exerts desired pharmacokinetic and pharmacodynamic properties.

6. The nanostructure for in vivo administration of claim 1, which is designed to function as a T1 MRI contrast agent that exhibits a bright signal in magnetic resonance imaging (MRI),
wherein animal studies can be used to analyze the in vivo behavior of nanostructures for in vivo administration or pharmaceutical compositions containing them, depending on the site of application and/or route of administration; and/or
it enables quality control to ensure that the nanostructures or pharmaceutical compositions are manufactured to a uniform quality to have the desired in vivo behavior.

7. The nanostructure for in vivo administration of claim 1, wherein the nanostructure can be finely tuned for physiological conditions, individual patient requirements, disease specifications, and/or intended route of administration, utilizing a wide variety of biological information data.

8. The nanostructure for in vivo administration of claim 1, wherein the average molecular weight of the dextran molecules in the dextran spherical core is 10,000 Da or less, the molecular weight of the spherical core formed by cross-linking the dextran molecules is 35,000 Da or less, and the hydrated diameter of the nanostructure is 10 nm or less, thereby to have a hydrodynamic diameter and molecular weight smaller than the renal clearance cut-off size, and to exhibit colloidal stability.

9. The nanostructure for in vivo administration of claim 1, wherein the hydration of hydrophilic functional groups exposed on the surface of the dextran spherical core improves colloidal stability in biological fluids.

10. The nanostructure for in vivo administration of claim 1, wherein the crosslinker-derived hydrophilic functional group to which the iron ion binds is a terminal functional group of the crosslinker or a modified functional group thereof.

11. The nanostructure for in vivo administration of claim 1, wherein some or all of the crosslinker-derived functional groups on the surface of the dextran spherical core are carboxylic acid or carboxylate groups.

12. The nanostructure for in vivo administration of claim 1, wherein it is stable in plasma without aggregation or free iron leaching, is not metabolized or degraded in the body after in vivo administration, and can be collected and reused via urine.

13. The nanostructure for in vivo administration of claim 1, wherein it circulates in the cerebral cardiovascular system.

14. The nanostructure for in vivo administration of claim 1, wherein after in vivo administration, it is absorbed into the blood circulation and excreted through the kidneys into the urine without extravasation through the blood vessel wall.

15. The nanostructure for in vivo administration of claim 1, wherein upon administration into a lymphatic vessel, articular cavity, or intrathecal space, it is absorbed into the blood circulation and excreted in the urine via the kidneys.

16. The nanostructure for in vivo administration of claim 1, wherein it is a circulating nanostructure that is not removed by the liver when injected into a vein, but is excreted through the kidneys into the urine after circulation.

17. The nanostructure for in vivo administration of claim 1, wherein it does not accumulate in tissues or organs, after in vivo administration and is excreted via renal clearance.

18. The nanostructure for in vivo administration of claim 1, wherein the overall size and overall charge of the nanostructure can be adjusted to impart the desired blood circulation time and renal excretion pharmacokinetics, by controlling the molecular weight of the dextran, the length of the dextran main chain, the type of crosslinker at crosslinking, the amount and rate of administration of the crosslinker during the synthesis reaction, and at least one of the additional chemical functional group modifications.

19. The nanostructure for in vivo administration of claim 1, wherein the shell of divalent or trivalent iron ions coordinationally bonded to crosslinker-derived hydrophilic groups on the surface of the spherical core is not coated with an additional chemical, but is exposed on its own, facilitating access of water molecules, which is more favourable for accelerating water proton relaxation of the nuclear spin in hydrogen atom.

20. The nanostructure for in vivo administration of claim 1, wherein it can act as a contrast agent during magnetic resonance imaging (MRI) to visualize microvessels, ureters, liver, spleen, lymphatic vessels, joint cavities, spinal cord cavities, and/or anatomical structures in vivo.

21. A pharmaceutical composition comprising a nanostructure for in vivo administration that after in vivo administration, is excreted in the urine via the kidneys without being phagocytosed by macrophages and/or metabolized,
wherein the nanostructure is a nanostructure for in vivo administration in any one of claims 1 to 20, comprising
(i) a spherical core formed by crosslinking two or three dextran molecules with an average molecular weight of 10,000 Da or less using a crosslinker and (ii) a discontinuous shell with divalent or trivalent iron ions coordinationally bonded to crosslinker-derived hydrophilic groups on the surface of the spherical core; and
(iii) a mass ratio of dextran to iron ranging from 100 : 2 to 100 : 10, with a crosslinker substitution ratio adjusted to 10% to 50% of the total number of functional groups on the dextran, while 20% to 50% of the crosslinker does not participate in crosslinking at the terminal end, resulting in the nanostructure having the charge ranging from -20 mV to 0 mV;
(iv) wherein only 20% to 80% of the crosslinker-derived hydrophilic groups externally exposed on the surface of the spherical core bind iron ions, while the remainder do not bind iron ions and are exposed to an aqueous environment.

22. The pharmaceutical composition of claim 21, wherein the nanostructure for in vivo administration is excreted in the urine via the kidneys without leaching iron and without being phagocytosed by macrophages, thereby not causing iron accumulation in the joints, which is a cause of joint disease.

23. The pharmaceutical composition of claim 21, wherein the nanostructures for in vivo administration are used as MRI contrast agents to realize imaging of vascular structures and morphology, analysis of blood flow and hemodynamic information, and/or imaging of the cardiovascular system, cerebrovascular system, lymphatic system, musculoskeletal system, and/or cranial spinal nervous system.

24. The pharmaceutical composition of claim 21, wherein the nanostructure for in vivo administration exerts a contrasting effect with surrounding tissues and, depending on the concentration distributed in the body fluid, the magnitude of the signal in the body fluid as seen in magnetic resonance imaging (MRI) varies, determining the distribution of tissues in time series.

25. The pharmaceutical composition of claim 21, wherein it also exerts T1 contrast effect when used in combination with an iodinated X-ray contrast agent.

26. The pharmaceutical composition of claim 21, wherein it contains the nanostructure for in vivo administration as an MRI contrast agent and is used in MR angiography, MR arthrography, MR cisternography, MR myelography, MR lymphangiography, MR cholangiopancreatography, or brain, abdominal MRI imaging.

27. The pharmaceutical composition of claim 21, wherein it is used as an MRI contrast agent; and/or as a drug carrier or as an adsorbent for collecting information in tissue, blood, or lymphatic fluid.

28. The pharmaceutical composition of claim 21, wherein the average molecular weight of the dextran is adjusted so that it is used as an intravenous or lymphatic injectable with a controlled concentration and viscosity.

29. The pharmaceutical composition of claim 21, wherein the pharmaceutical composition is prepared by a method comprising the following steps:
1^{st} step of preparing an aqueous solution of dextran;
2^{nd} step of adding an alkaline aqueous solution and an epoxide at room temperature;
3^{rd} step of adding two or more polyvalent amines at room temperature, to generate crosslinked dextran-based nanoparticles with terminal amine groups;
4^{th} step of precipitate the product;
5^{th} step of redispersing the product in water;
optionally, 6^{th} step of recovering the crosslinked dextran-based nanoparticles with terminal amine groups by dialysis;
7^{th} step of treating an organic acid anhydride to the crosslinked dextran-based nanoparticles crosslinked by a polyvalent amine group and having terminal amine groups, to substitute some or all of the terminal amine groups with carboxylic acid groups and/or carboxylate groups;
optionally, 8^{th} step of purifying a solution of crosslinked dextran-based nanoparticles with carboxylic acid and/or carboxylate functional groups, to prepare water dispersible, crosslinked dextran-based nanoparticles;
9^{th} step of adding an aqueous solution of an iron precursor to the water dispersible, crosslinked dextran-based nanoparticles prepared in the preceding step, to form a shell comprising divalent to trivalent iron ions; and
optionally, 10^{th} step of purifying or concentrating the nanostructures formed in the preceding step by ultrafiltration; and
wherein the obtained nanostructures for in vivo administration are in the form of colloids dispersed in water, which can be used as an injectable solution on their own without any further processing, satisfying sterility and non-pyrogenicity, and/or can be made isotonic without any excipients, depending on the degree of concentration.

30. The pharmaceutical composition of claim 21, wherein the nanostructure for in vivo administration is linked, as an MRI contrast agent, to a drug carrier comprising a target antigen binding site.
